# EUROPEAN PATENT SPECIFICATION

(11) **EP 0 529 076 B1**
(45) Date of publication and mention of the grant of the patent: **06.12.1995**
(21) Application number: 91908180.2
(22) Date of filing: 25.04.1991
(51) Int. Cl.: C07K 14/71, C12N 15/00, C12N 1/20

(54) **PROTEINS WITH FIBROBLAST GROWTH FACTOR RECEPTOR ACTIVITY**
PROTEIN MIT FIBROBLASTEN-WACHSTUMSFAKTOR-REZEPTORAKTIVITÄT
PROTEINES PRESENTANT UNE ACTIVITE DE RECEPTEUR DE FACTEUR DE CROISSANCE DE FIBROBLASTE

(30) Priority: 27.04.1990 JP 113146/90; 31.07.1990 JP 204438/90; 14.09.1990 JP 245256/90; 28.12.1990 JP 415801/90
(43) Date of publication of application: 03.03.1993
(73) Proprietor: Takeda Chemical Industries, Ltd., Chuo-ku, Osaka (JP)
(72) Inventor: IGARASHI, Koichi, 66-3, Shimogamo-Miyazaki-cho, kyoto 606 (JP); SENOO, Masaharu, B-212, 744-1,, Okayama-shi, OKAYAMA 703 (JP); WATANABE, Tatsuya, 50-1,, Osaka 565 (JP)
(74) Representative: von Kreisler, Alek, Dipl.-Chem.
(86) International application number: JP9100557
(87) International publication number: WO9117183

(56) References cited:
- Science, vol. 245, 7 July 1989, P.L. Lee et al.:"Purification and complementary DNA cloning of a receptor for basic fibroblast growth factor", pages 57-60, see the whole article
- Proc. Natl. Acad. Sci, vol. 86, July 1989, E.B. Pasquale et al.: "Identification of a developmentally regulated protein-tyrosine kinase by using anti-phosphotyrosine antibodies to screen a cDNA expression library", pages 5449-5453, see the whole article
- Proc. Natl. Acad. Sci, vol. 87, August 1990, Y. Hattori et al.: "K-sam, an amplified gene in stomach cancer, is a member of the heparin-binding growth factor receptor genes", pages 59-83-5987,see the whole article
- The EMBO Journal, vol. 9, no, 9, September 1990, Oxford University Press C.A. Dionne et al.: "Cloning and expression of two distinct high-affinity receptors cross-reacting with acidic and basic fibroblast growth factors", pages2685-2692, see the whole article
- Science, vol. 251, 4 January 1991, T. Miki et al.: "Expression of cDNA cloning of the KGF receptor by creation of a transforming autocrine loop", pages 72-75, see the whole article

## Description

### FIELD OF THE INVENTION

The present invention relates to a water-soluble mutein of an animal protein which acts as a receptor for a fibroblast growth factor (hereinafter also referred to as FGF) protein, a recombinant DNA coding for the mutein and a technique for producing the mutein.

### BACKGROUND OF THE INVENTION

FGFs include acidic fibroblast growth factors (hereinafter also referred to as aFGFs) having a molecular weight of about 16,000 and an isoelectric point of 5 to 7 which are localized in hypothalami, brains and retinas, and basic fibroblast growth factors (hereinafter also referred to as bFGFs) having a molecular weight of about 17,000 and an isoelectric point of 8 to 10 which widely occur in various tissues and organs. They both are characterized by strong heparin-binding ability, and at first were isolated as factors exhibiting strong growth promoting action on fibroblasts. It has thereafter become clear that they exhibit growth promoting action on almost all mesoderm-derived cells. In particular, they are generally known as growth promoting factors or angiogenic factors for endothelial cells. Most cancer cells are said to produce bFGFs, which promote growth of cancer cells themselves.

It is considered that the mutual interaction between FGFs and FGF receptors existing on the surfaces of cells is essential to the action of FGFs on various cells. Further, it is suggested that the aFGF and the bFGF possibly bind to a common receptor. However, this is not clear in all cases.

As described above, although FGF receptors play an important role in various cells, the facts are not clear as to the properties of FGF receptors as proteins and their genes. There is no known report that an FGF receptor has been produced by recombinant DNA technology. If the FGF receptors can be produced in large amounts by using recombinant DNA technology, they can be used as pharmaceuticals, such as antitumor agents. In particular, one goal has been to establish a mass production method for the proteins which act as a receptor for human FGF.

In general, proteins of closely related animals have high homology in their amino acid sequences, and most of the different amino acid portions are derived by one point mutation of codons of their genes. The present inventors deduced that a portion of the DNA sequence of a chicken FGF receptor gene would be very similar to the DNA sequence of a gene coding for a protein acting as a receptor for human FGF. Based on this idea, the present inventors synthesized a DNA having the same nucleotide sequence with a portion of the chicken FGF receptor gene as a probe, and cloned a gene coding for a protein which acts as a receptor for human FGF from a human cell by using this DNA. Then, the present inventors constructed a recombinant DNA containing the above gene and cultivated a transformant transformed with the above recombinant DNA. As a result, the present inventors discovered that a protein acting as a receptor for human FGF was produced.

Further, the present inventors discovered that a water-soluble mutein of the animal protein having this activity for the FGF protein advantageously received the FGF protein.

The present inventors further investigated based on this information, and consequently completed the present invention.

### SUMMARY OF THE INVENTION

The present invention provides:
(1) a water-soluble mutein of an animal protein having activity as a receptor for a fibroblast growth factor (FGF) protein,
(2) a recombinant DNA having a nucleotide sequence coding for the water-soluble mutein of the above item (1),
(3) a vector containing the recombinant DNA of the above item (2),
(4) a transformant transformed with the vector of the above item (3), and
(5) a process for producing the water-soluble mutein of the above item (1) which comprises cultivating the transformant of the above item (4) in a culture medium.

### BRIEF DESCRIPTION OF THE DRAWINGS

Fig. 1 shows a portion of a nucleotide sequence of a cDNA coding for a protein having activity as a receptor for human FGF, the protein being produced in Example 1, and an amino acid sequence deduced therefrom;
Fig. 2 shows restriction enzyme maps of the cDNA of an FGF receptor obtained in Example 2;
Fig. 3 shows a nucleotide sequence of the cDNA of plasmid pTB1228 obtained in Example 2, and an amino acid sequence to be encoded, in the lower row;
Fig. 4 shows the nucleotide sequence of the cDNA of plasmid pTB1229 obtained in Example 2, and the amino acid sequence to be encoded, in the lower row;
Fig. 5 is a schematic representation showing the construction of plasmid pTB1283 obtained in Example 3;
Fig. 6 is a schematic representation showing the construction of plasmid pTB1284 obtained in Example 3;
Fig. 7 shows a nucleotide sequence of the cDNA of plasmid pTB1284 obtained in Example 3, and the amino acid sequence to be encoded, in the lower row;
Fig. 8 shows a nucleotide sequence of the cDNA of plasmid pTB1283 obtained in Example 3, and the amino acid sequence to be encoded, in the lower row;
Fig. 9 is a schematic representation showing the construction of plasmid pTB1289 obtained in Example 4;
Fig. 10 is a schematic representation showing the construction of plasmid pTB1290 obtained in Example 4;
Fig. 11 shows a nucleotide sequence of the cDNA of plasmid pTB1289 obtained in Example 4, and an amino acid sequence of an extracellular domain of an FGF receptor produced thereby;
Fig. 12 shows a nucleotide sequence of the cDNA of plasmid pTB1290 obtained in Example 4, and an amino acid sequence of an extracellular domain of an FGF receptor produced thereby;
Fig. 13 is a schematic representation showing the construction of plasmid pTB1313 obtained in Example 3 (2);
Fig. 14 is a schematic representation showing the construction of plasmid pTB1315 obtained in Example 4 (2); and
Fig. 15 is a graph which shows purification by a Q-Sepharose column of FGF receptor extracellular domain produced in E. coli.

### DESCRIPTION OF THE PREFERRED EMBODIMENTS

In the present invention, the FGF proteins include acidic FGFs, basic FGFs and muteins thereof. In particular, the basic FGFs and the muteins thereof are preferably used.

As the FGFs, mammal-derived FGFs are preferably used. Examples of the mammals include humans, monkeys, pigs, cattle, sheep and horses.

The muteins of the FGFs include a mutein in which at least one FGF-constituent amino acid is deleted from the FGF, a mutein in which at least one FGF-constituent amino acid is substituted for another amino acid, and a mutein in which at least one amino acid is added to the FGF, each mutein having FGF activity.

Specific examples of the FGF proteins include, for example, proteins disclosed in European Patent Publication Nos. 237,966, 281,822, 326,907 and 394,951.

The activity as a receptor for the FGF proteins includes the property of binding to the extracellular domains of the FGF proteins, and refers to activation of portions corresponding to tyrosine-phosphorylation enzymes in the intracellular domains by structural change due to binding to the FGF proteins. It is generally considered that such action of tyrosine phosphorylation is closely related to cell differentiation and cell proliferation.

As proteins which specifically bind to FGF protein molecules, anti-FGF antibodies are considered. However, antibody molecules have no enzyme activity and are independent of cell differentiation and cell proliferation. Accordingly, the claimed proteins which act as receptors for the FGF proteins are essentially different from any anti-FGF antibodies.

The animal proteins which act as receptors for the FGF proteins, which are basic materials of the water-soluble muteins of the present invention, include human, chicken, mouse, frog and fish proteins. The animal proteins are hereinafter also referred to as mature FGF receptors.

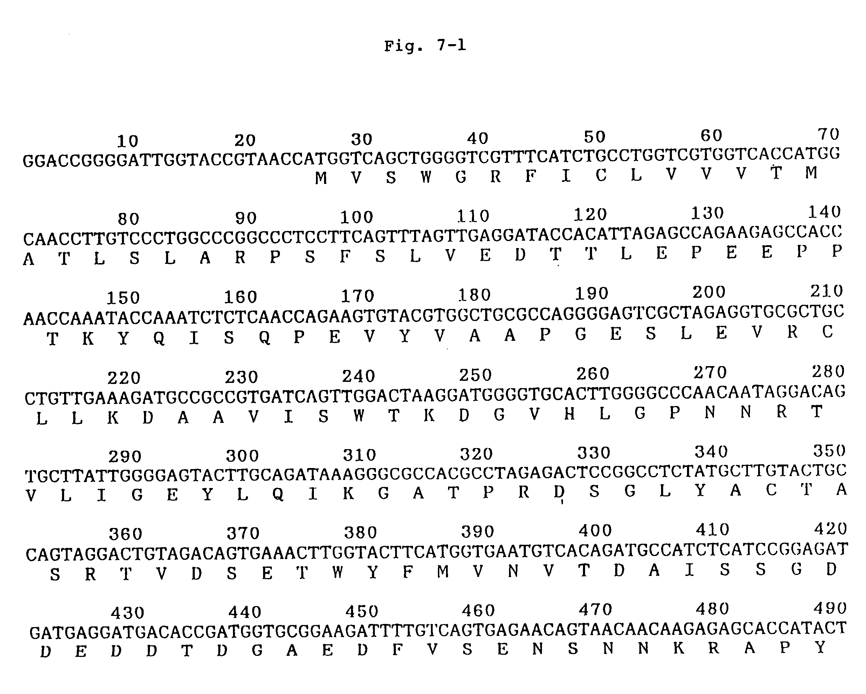

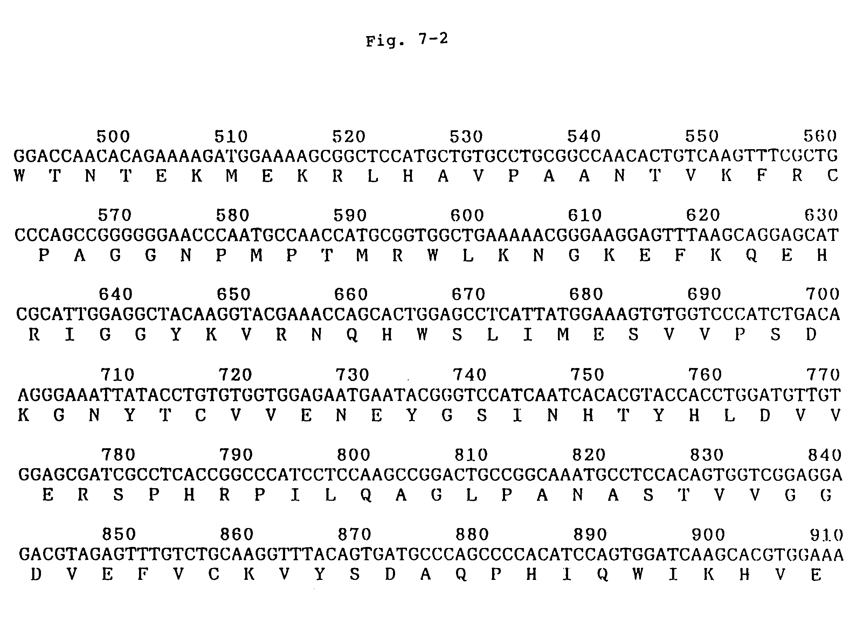

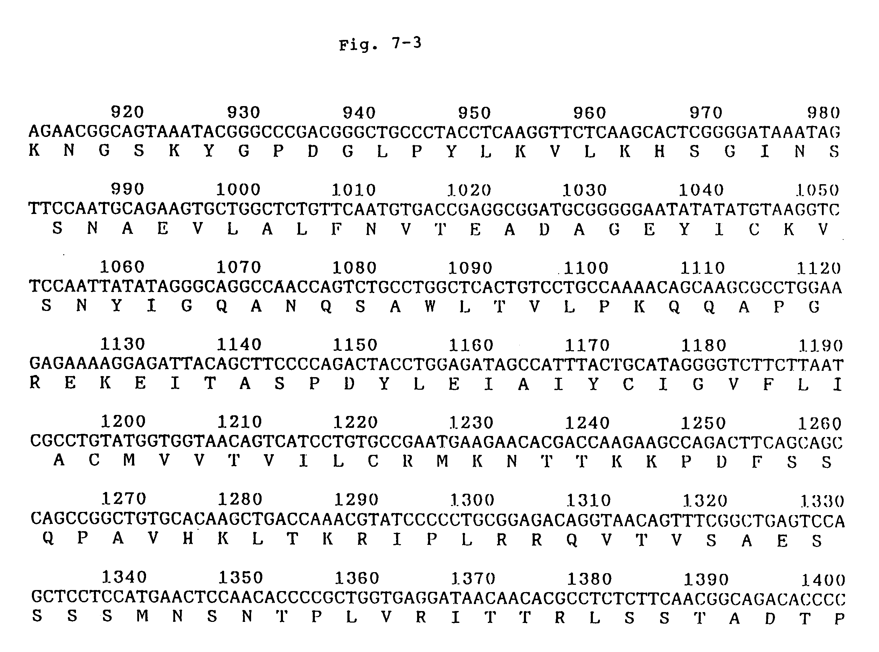

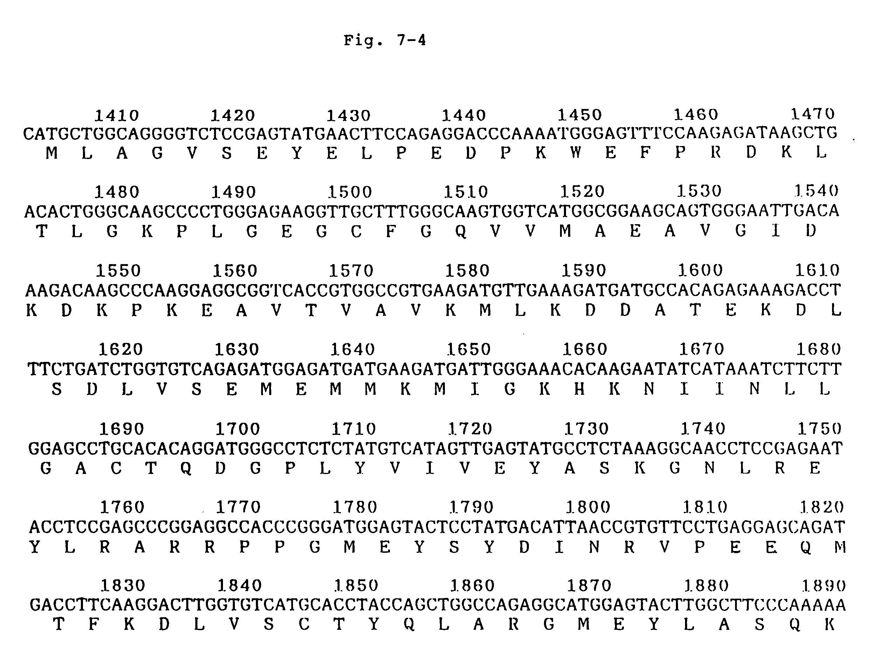

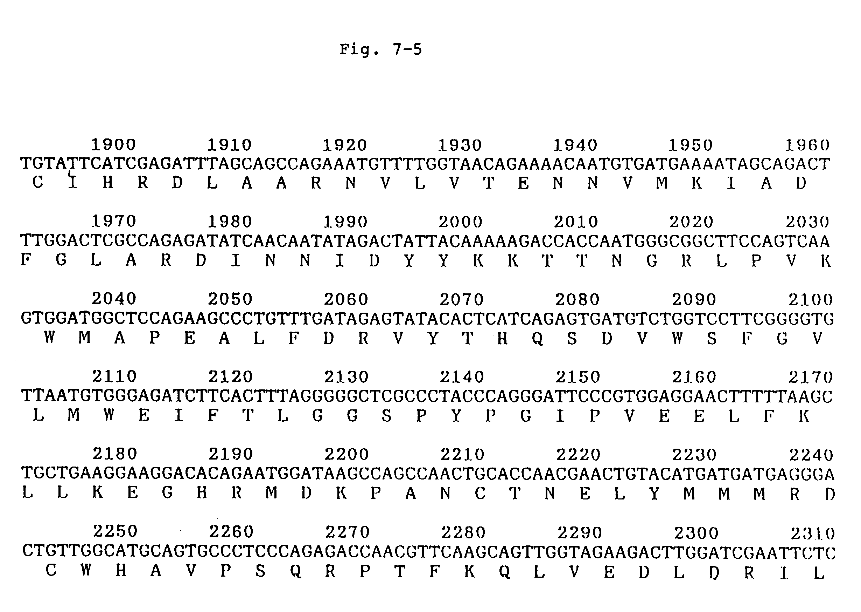

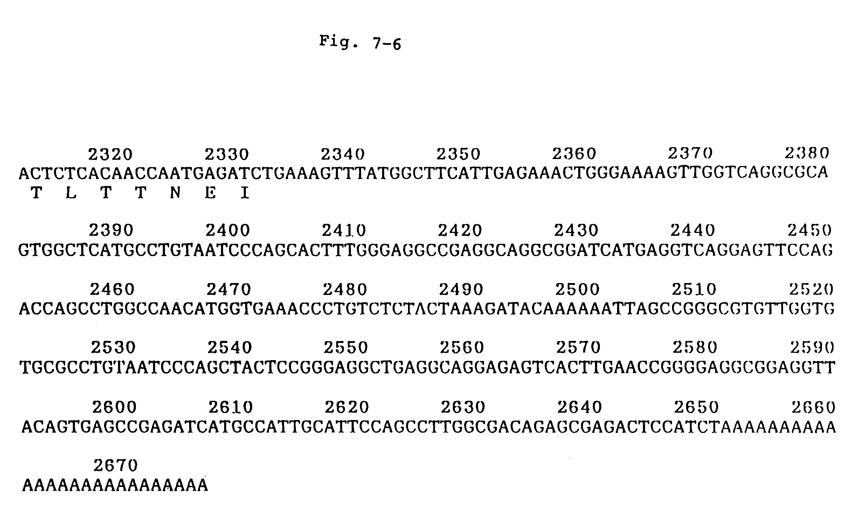

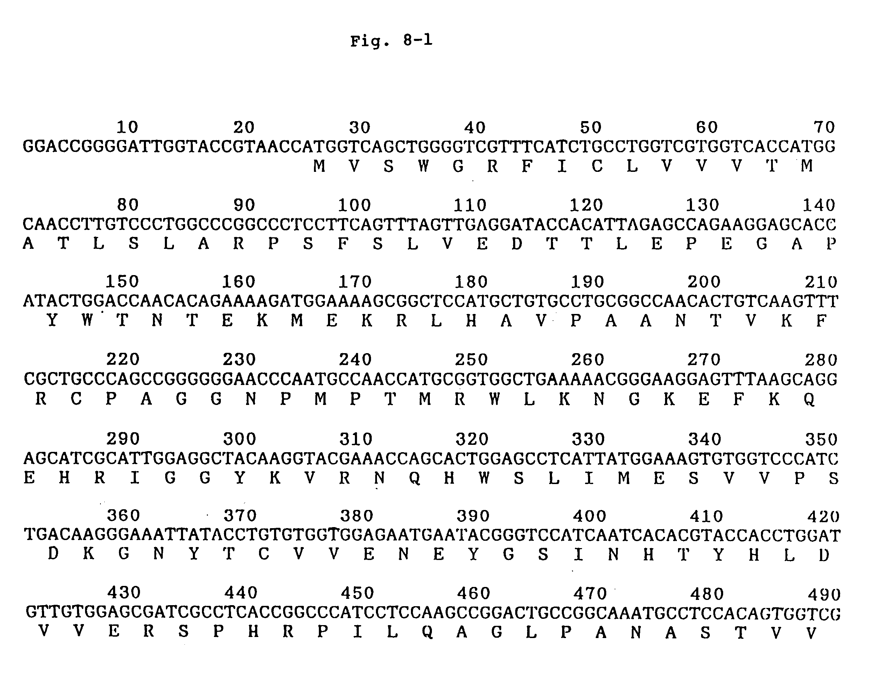

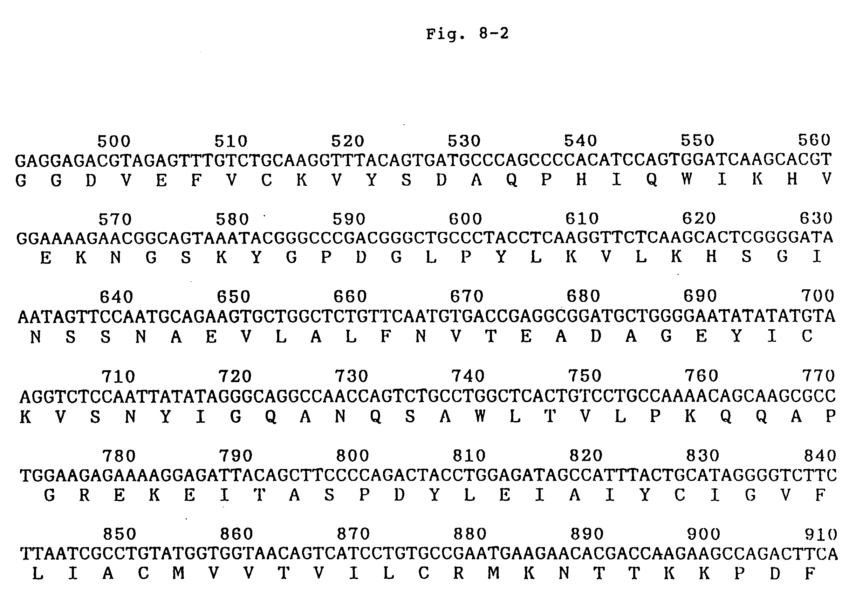

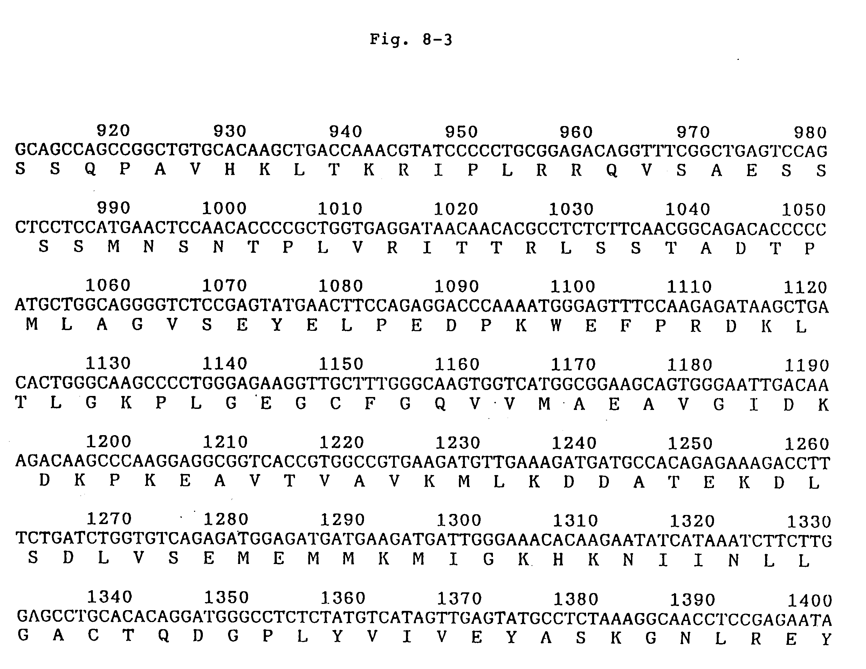

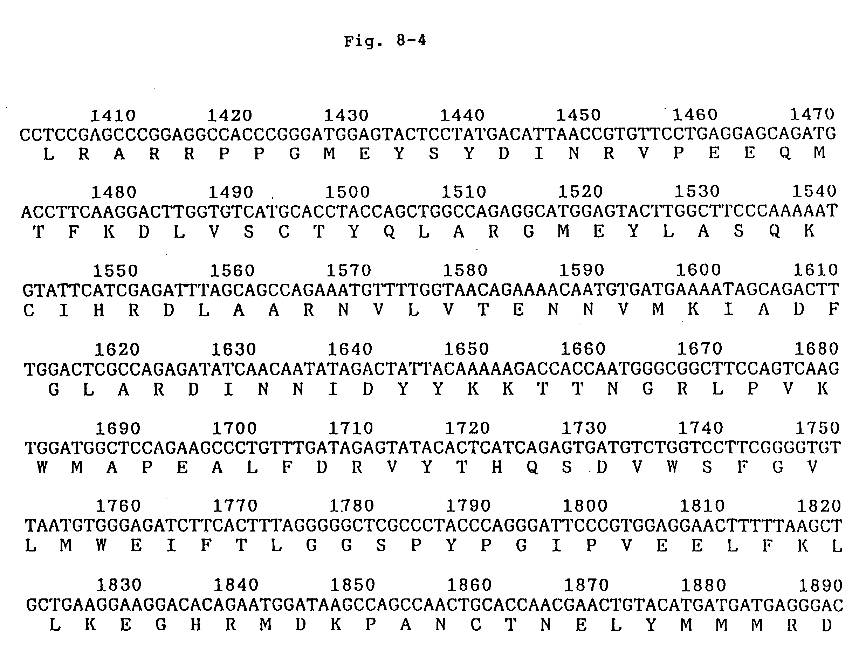

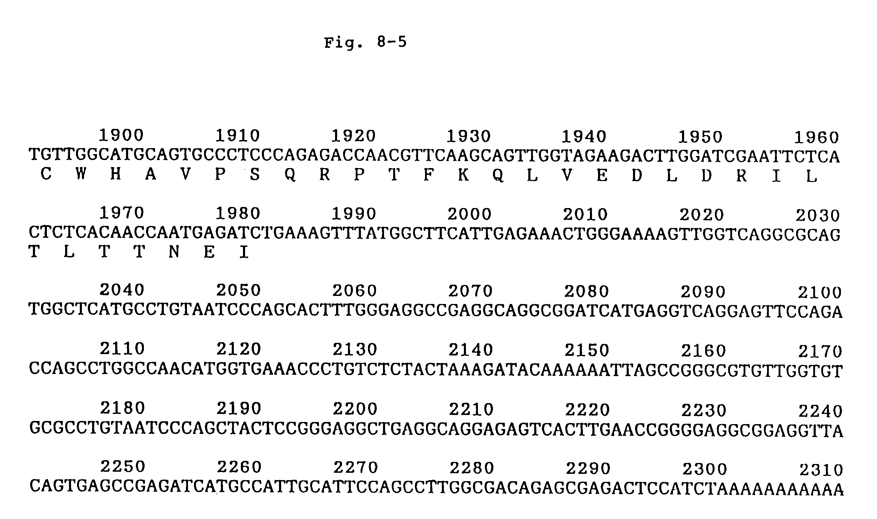

The human mature FGF receptors include a protein having an amino acid sequence formed by continuously connecting the amino acid sequence shown in Fig. 7, a protein having an amino acid sequence formed by continuously connecting the amino acid sequence shown in Fig. 8, a protein having an amino acid sequence described in Nucleic Acids Research 18, 1906 (1990), a protein having an amino acid sequence described as "flg" in The EMBO Journal 9, 2685 (1990), a protein having an amino acid sequence described as "bek" in Molecular and Cellular Biology 8, 5541-5544 (1988) and The EMBO Journal 9, 2685 (1990), and a protein having an amino acid sequence described as a protein expressing K-sam gene in Proc. Natl. Acad. Sci. USA 87, 4378-4382 (1990).

The chicken mature FGF receptors include a protein having an amino acid sequence described in Science 245, 57-60 (1989).

The mouse mature FGF receptors include a protein having an amino acid sequence described in Proc. Natl. Acad. Sci. USA 87, 4378-4382 (1990).

The water-soluble muteins of the present invention include muteins lacking at least one amino acid from the C-terminal side of the mature FGF receptor, and muteins lacking amino acid sequences corresponding to transmembrane domains of the mature FGF receptor.

The muteins lacking at least one amino acid from the C-terminal side of the mature FGF receptor include muteins lacking amino acid residues from the C-terminus to the transmembrane domains, namely portions corresponding to extracellular domains.

The extracellular domains may further lack 1 to 38 amino acid residues from the C-terminus thereof. Furthermore, the extracellular domains may lack up to 123 amino acid residues from the C-terminus thereof.

The muteins lacking amino acid sequences corresponding to transmembrane domains of the mature FGF receptors may further lack 1 to 11 amino acid residues from the C-terminus thereof.

The above-mentioned deleted muteins may further lack at least one amino acid from the N-terminus simultaneously.

The deletion from the N-terminus include deletion of 1 to 257 amino acid residues, and preferably 1 to 131 amino acid residues from the N-terminus. The number of the amino acid is understood to be counted from an amino acid next to a signal sequence of the N-terminus.

In order to produce the water-soluble muteins of the present invention, site-directed mutagenesis is employed in addition to the conventional genetic engineering technique. This mutagenesis, which is well known, is described in R. F. Lather and J. P. Lecoq, Genetic Engineering, Academic Press, p.31-50 (1983). Mutagenesis directed to oligonucleotide is described in M. Smith and S. Gillam, Genetic Engineering: Principles and Methods, Plenum Press, vol. 3, p.1-32 (1981). A structural gene coding for the mutein of the present invention is produced, for example, by the steps of:
(a) hybridizing a single stranded DNA comprising one strand of a structural gene of the mature FGF receptor with a mutagenic oligonucleotide primer,
(b) elongating the primer with DNA polymerase to form a mutational heteroduplex, and
(c) replicating this mutational heteroduplex.

The size of the oligonucleotide primer depends on conditions required for stable hybridization of the primer to a gene region to which mutation is to be introduced, and on limitations in currently available methods of oligonucleotide synthesis. The factors (for example, the whole size and the size of a mismatching portion for a mutation site) to be considered in designing the oligonucleotide used for mutagenesis directed by the oligonucleotide are described by M. Smith and S. Gillam in the above literature. In general, the whole length of the oligonucleotide is such a length that stable, unique hybridization at the mutation site is optimized, and the extensions from the mutation site to the 5'- and the 3'-termini are adjusted in size so as to be sufficient to prevent mutation editing due to the exonuclease of DNA polymerase.

The expression vector having the DNA containing the nucleotide sequence coding for the above-mentioned mature FGF receptor can be prepared, for example, by the steps of:
(i) isolating RNA coding for the mature FGF receptor,
(ii) synthesizing single stranded complementary DNA (cDNA) from the mRNA, followed by synthesis of double stranded DNA,
(iii) introducing the complementary DNA into a plasmid,
(iv) transforming a host with the recombinant plasmid thus obtained,
(v) cultivating the transformant thus obtained, and then isolating the plasmid containing the desired DNA from the transformant by an appropriate method such as colony hybridization using a DNA probe,
(vi) cutting out the desired cloned DNA from the plasmid, and
(vii) ligating the cloned DNA downstream from a promoter in the vehicle.

The above-mentioned cDNA can also be produced by chemical synthesis.

The RNA coding for the mature FGF receptors can be obtained from various FGF receptor-producing cells such as human endothelium-derived cells and human fibroblasts. The human fibroblasts include WI38 (ATCC No. CCL-75) and IMR90 (ATCC No. CCL-186). The above cells WI38 and IMR90 appear in Catalogue of Cell Lines & Hybridomas, 5th edition (1985), published by The American Type Culture Collection.

Methods for preparing the RNA from the mature FGF receptor-producing cells include the guanidine thiocyanate method [J. M. Chirgwin et al., Biochemistry 18, 5294 (1979)].

Using the RNA thus obtained as a template, cDNA is synthesized by use of reverse transcriptase, for example, in accordance with the method of H. Okayama et al. [Molecular and Cellular Biology 2, 161 (1982); ibid. 3, 280 (1983)]. The cDNA thus obtained is introduced into the plasmid.

The plasmids into which the cDNA is introduced include, for example, pBR322 [Gene 2, 95 (1977)], pBR325 [Gene 4, 121 (1978)], pUC12 [Gene 19, 259 (1982)], pUC13 [Gene 19, 259 (1982)], pUC118 and pUC119 [Methods in Enzymology 153, 3-11 (1987)], each derived from Escherichia coli, and pUB110 derived from Bacillus subtilis [Biochemical and Biophysical Research Communication 112, 678 (1983)]. However, any other plasmid can be used as long as it is replicable and maintained in the host.

Methods for introducing the cDNA into the plasmid include, for example, the method described in T. Maniatis et al., Molecular Cloning, Cold Spring Laboratory, p.239 (1982).

As the plasmid into which the above cDNA is introduced, a plasmid may be used which is obtained by using the cDNA library in which E. coli x1776 prepared by introducing cDNA synthesized from human normal diploid cell mRNA into the pCD vector [H. Okayama et al., Molecular Cell Biology 3, 280 (1983)] is used as a host. The above cDNA library is available from Dr. Okayama, Research Institute for Microbial Diseases, Osaka University.

The plasmid thus obtained is introduced into the appropriate host cells such as Escherichia and Bacillus.

Examples of Escherichia described above include E. coli K12DHl [Proc. Natl. Acad. Sci. USA 60, 160 (1968)], M103 [Nucleic Acids Research 9, 309 (1981)], JA221 [Journal of Molecular Biology 120, 517, (1978)], HB101 [Journal of Molecular Biology 41, 459 (1969)] and C600 [Genetics 39, 440 (1954)].

Examples of Bacillus described above include Bacillus subtilis MI114 [Gene 24, 255 (1983)] and 207-21 [Journal of Biochemistry 95, 87 (1984)].

The methods of transformation include, for example, the calcium chloride method and the calcium chloride/rubidium chloride method described in T. Maniatis et al., Molecular Cloning, Cold Spring Harbor Laboratory, p.249 (1982).

The desired clones are selected from the thus-obtained transformants by using known methods such as the colony hybridization method [Gene 10, 63 (1980)] and the DNA nucleotide sequence determination method [Proc. Natl. Acad. Sci. USA 74, 560 (1977)].

Thus, the microorganisms are obtained each of which carries the vector having the DNA containing the nucleotide sequence coding for the cloned mature FGF receptor.

Then, the plasmids are isolated from the microorganisms.

Isolation methods include the alkali methods [H. C. Birmboim et al., Nucleic Acids Research 1, 1513 (1979)].

The plasmid having the DNA containing the nucleotide sequence coding for the cloned mature FGF receptor can be used as it is, or cut out by digestion with a restriction enzyme if desired.

The cloned gene is ligated downstream from the promoter in a vehicle (vector) suitable for expression, whereby the expression vector can be obtained.

The vectors include the above plasmids derived from E. coli (such as pBR322, pBR325, pUC12, pUC13, pUC118 and pUC119), plasmids derived from B. subtilis (such as pUB110, pTP5 and pC194), plasmids derived from yeast (such as pSH19 and pSH15), bacteriophages such as λphage, and animal viruses such as retroviruses and vaccinia viruses.

The gene has ATG as a translation initiating codon at the 5'-terminus thereof and may have TAA, TGA or TAG as a translation terminating codon at the 3'-terminus. Further, in order to express the gene, the promoter is ligated upstream therefrom. As the promoter used in the present invention, any promoter is available as long as it is suitable for expression corresponding to the host used for the gene expression.

When the host used for transformation is Escherichia, it is preferable to use a T7 promoter, a trp promoter, a lac promoter, a recA promoter, a λpL promoter, a lpp promoter or the like. When the host is Bacillus, it is preferable to use a SPO1 promoter, a SPO2 promoter, a penP promoter or the like. When the host is yeast, it is preferable to use a PHO5 promoter, a PGK promoter, a GAP promoter, an ADH promoter or the like. In particular, it is preferable that the host is Escherichia and the promoter is the trp promoter or the T7 promoter.

When the host is an animal cell, an SV40-derived promoter, a retrovirus promoter or the like can be used.

By using a vector containing the DNA thus constructed, the transformant is prepared.

Examples of the host cells include Escherichia, Bacillus, yeast and animal cells.

Specific examples of Escherichia and Bacillus described above include the strains described above.

Examples of the yeast described above include Saccharomyces cerevisiae AH22R, NA87-11A and DKD-5D.

Examples of the animal cells include monkey cell COS7, Vero, Chinese hamster ovary cell (CHO), mouse L cell and human FL cell.

The Escherichia described above is transformed, for example, according to the methods described in Proc. Natl. Acad. Sci. USA, 69, 2110 (1972), Gene, 17, 107 (1982) and the like.

The Bacillus is transformed, for example, according to the methods described in Molecular & General Genetics, 168, 111 (1979) and the like.

The yeast is transformed, for example, according to the method described in Proc. Natl. Acad. Sci. USA, 75, 1929(1978).

The animal cells are transformed, for example, according to the method described in Virology, 52, 456 (1973).

Thus, the transformant transformed with the vector containing the DNA is obtained.

When the Escherichia or Bacillus transformant is cultivated, a liquid medium is particularly suitable as a medium for cultivation. Carbon sources, nitrogen sources, inorganic compounds and others necessary for growth of the transformants are contained therein. The carbon sources include, for example, glucose, dextrin, soluble starch and sucrose. The nitrogen sources include inorganic or organic materials such as ammonium salts, nitrates, corn steep liquor, peptone, casein, meat extracts, soybean meal and potato extract solution. The inorganic compounds include, for example, calcium chloride, sodium dihydrogenphosphate and magnesium chloride. Further, yeast, vitamins, growth promoting factors and so on may be added thereto.

The pH of the medium is preferably from about 6 to 8.

As the medium used for cultivation of Escherichia, it is preferable to use, for example, M9 medium containing glucose and Casamino Acids (Miller, Journal of Experiments in Molecular Genetics, 431-433, Cold Spring Harbor Laboratory, New York, 1972). In order to make the promoter act efficiently, a drug such as 3β-indolylacrylic acid may be added thereto, if necessary.

When the host is Escherichia, the cultivation is usually carried out at about 15 to 43^{o}C for about 3 to 24 hours, with aeration or agitation if necessary.

When the host is Bacillus, the cultivation is usually carried out at about 30 to 40^{o}C for about 6 to 24 hours, with aeration or agitation if necessary.

When the yeast transformant is cultivated, there is used, for example, Burkholder minimum medium [K. L. Bostian et al., Proc. Natl. Acad. Sci. USA, 77, 4505 (1980)] as the culture medium. The pH of the medium is preferably adjusted to from about 5 to 8. The cultivation is usually carried out at about 20 to 35^{o}C for about 24 to 72 hours, with aeration or agitation if necessary.

When the animal cell transformant is cultivated, preferred examples of the culture media include MEM medium containing about 5 to 20% fetal calf serum [Science, 122, 501 (1952)], DMEM medium [Virology, 8, 396 (1959)], RPMI1640 medium [Journal of the American Medical Association, 199, 519 (1967)] and 199 medium [Proceeding of the Society for the Biological Medicine, 73, 1 (1950)]. The pH is preferably from about 6 to 8. The cultivation is usually carried out at about 30 to 40^{o}C for about 15 to 60 hours, with aeration or agitation if necessary.

The mutein of the present invention can be isolated and purified from the cultures described above, for example, by the following method.

When the mutein of the present invention is extracted from the cultivated cells, the cells are collected by methods known in the art after cultivation. Then, the collected cells are suspended in an appropriate buffer solution containing a protein denaturant such as guanidine hydrochloride to extract the desired mutein out of the cells. There is also suitably used the method that the cells are disrupted by ultrasonic treatment, lysozyme and/or freezing-thawing, followed by centrifugation to obtain the mutein of the present invention. In particular, the method using lysozyme in combination with ultrasonic treatment is preferred.

In order to purify the mutein of the present invention from a supernatant of the culture, suitable combinations of known separating and purifying methods per se can be used. These known separating and purifying methods include methods utilizing a solubility such as salt precipitation and solvent precipitation, methods mainly utilizing a difference in molecular weight such as dialysis, ultrafiltration, gel filtration chromatography and SDS-polyacrylamide gel electrophoresis, methods utilizing a difference in electric charge such as ion-exchange column chromatography, methods utilizing specific affinity such as affinity chromatography, methods utilizing a difference in hydrophobicity such as reverse-phase high performance liquid chromatography and methods utilizing a difference in isoelectric point such as isoelectro focusing electrophoresis.

The muteins of the present invention thus obtained can also be dialyzed and lyophilized to form dried powders. When serum albumin is added to the muteins of the present invention for storage, the muteins are preferably prevented from being adsorbed by containers.

Thus, the substantially pure water-soluble muteins of the present invention are obtained. The substantially pure water-soluble muteins of the present invention include muteins having a protein content of 95% (w/w) or more, and preferably muteins having a protein content of 98% (w/w) or more.

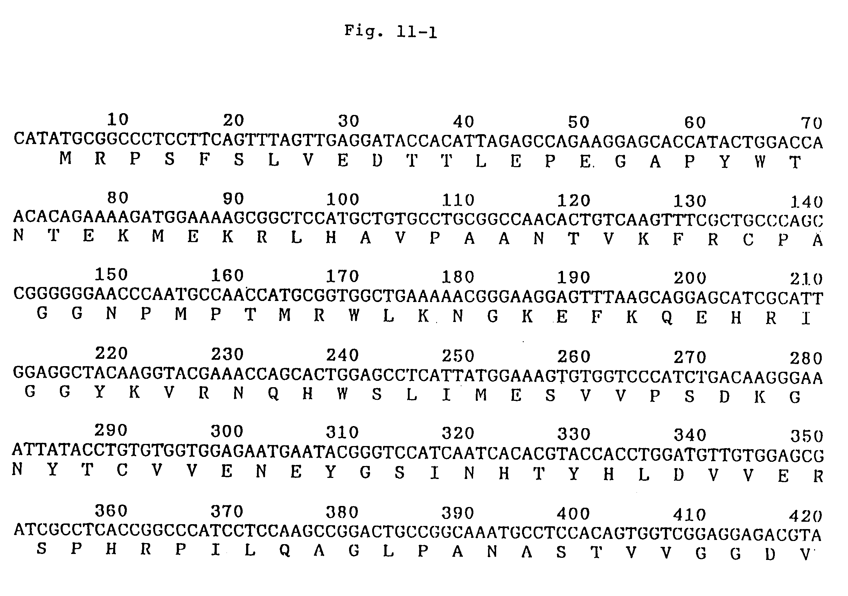

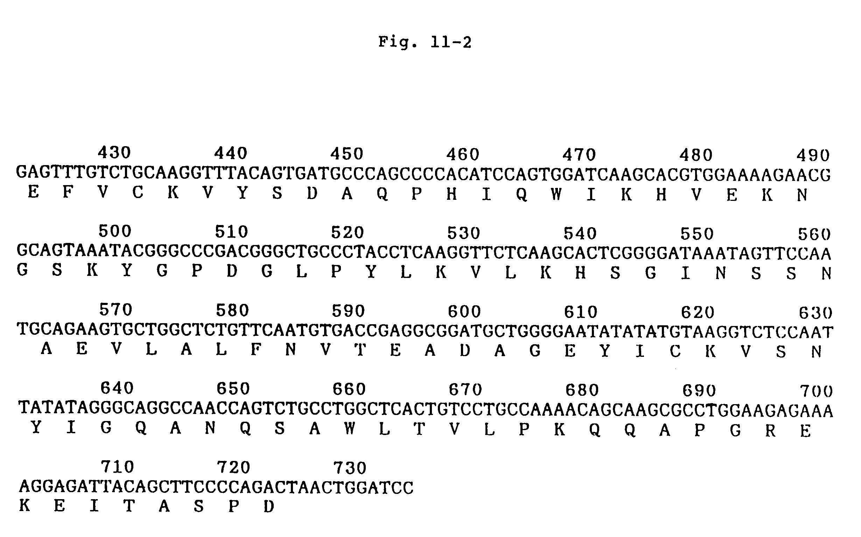

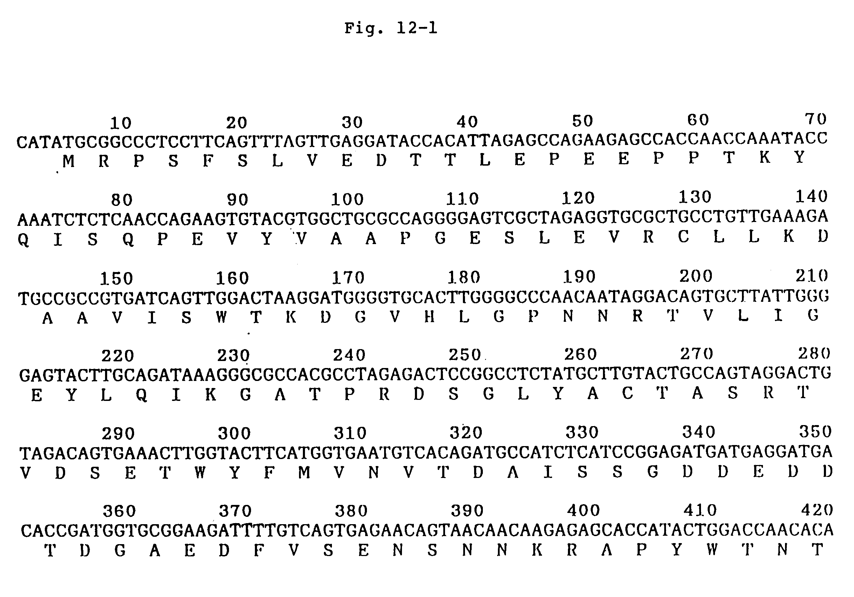

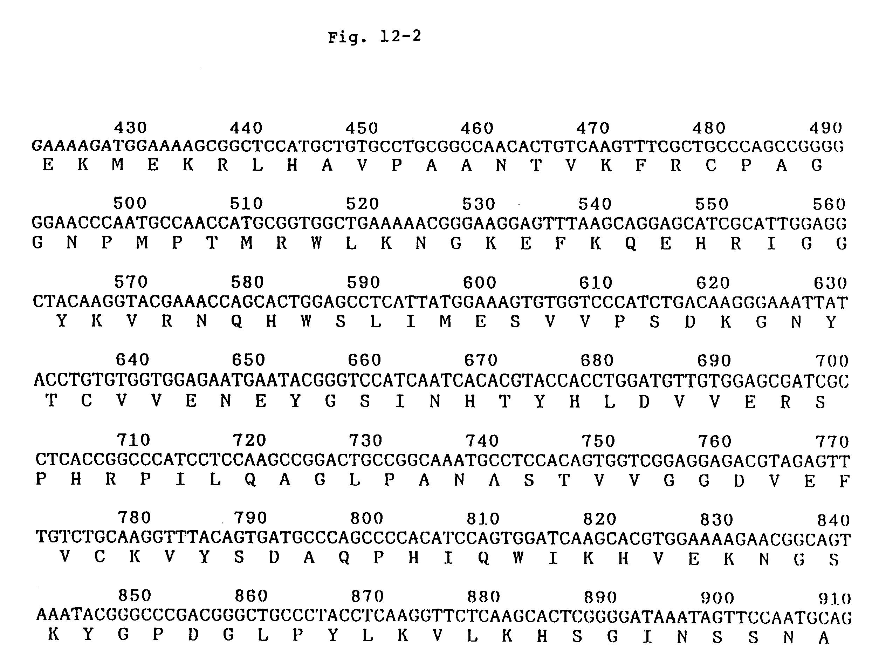

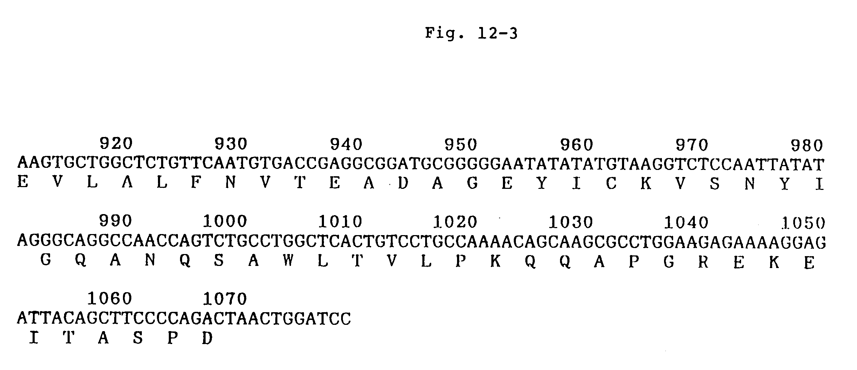

Examples of the muteins of the present invention obtained by recombinant DNA technology include a protein containing a polypeptide represented by an amino acid sequence shown in Fig. 11, and a protein containing a polypeptide represented by an amino acid sequence shown in Fig. 12. The above protein may have Met at the N-terminus thereof. To the C-termini of the water-soluble muteins of the present invention, amino acid sequences corresponding to the transmembrane domains or the intracellular domains may be added (the resulting polypeptide is hereinafter also referred to as amino acid adducts). Examples of such adducts include a polypeptide containing an amino acid sequence shown in Fig. 7, and a polypeptide containing an amino acid sequence shown in Fig. 8. The above polypeptides can be produced, for example, by preparing cDNAs by the methods described above, introducing the cDNAs into plasmids to prepare transformants, cultivating the transformants and purifying the products.

The activity of the thus-obtained water-soluble muteins or amino acid adducts of the present invention can be assayed based on the binding effect of FGFs to cells.

The cells transfected or transformed with the recombinant DNA of the present invention can produce a large amount of the muteins of the present invention, even in various cells which essentially produce only a small amount of the FGF receptors or do not produce them at all.

The expression plasmids containing the genes coding for the water-soluble muteins of the present invention can allow various cells to produce the muteins of the present invention by introducing the plasmids into the cells, so that the water-soluble muteins of the present invention can be produced in large amounts.

To the water-soluble muteins of the present invention, sugar chains may be added. The sugar chains may be any one found in known glycosylated proteins. Examples thereof include N-acetyl glycosamine, N-acetyl galactosamine, mannose, galactose, fucose and cyalic acid. The number of the sugar chains added to the muteins is at least one, and preferably 10 to 20.

Using the thus-obtained water-soluble muteins of the present invention or the amino acid adducts thereof, the FGF molecules can be prevented from binding to the FGF receptors naturally existing on the cell surfaces. The cell proliferation depending on FGF is inhibited by such prevention. For this reason, they can be used as therapeutic agents for multiple endocrine neoplasia, prostatic hypertrophy, diabetic retinitis or cancer. Their toxicity is low.

The muteins of the present invention or the amino acid adducts thereof can be produced by transfecting or transforming various animal cells (such as lymphocytic cells) originally free from the FGF receptors with the DNAs of the present invention. These cells can therefore be proliferated, subcultured, cell lines established or cloned in vitro for a long period of time by cultivation in FGF-containing culture media.

The above-mentioned animal cells of which proliferation becomes possible by using the DNAs of the present invention are cultivated in large amounts, whereby various useful substances essentially produced by these cells can be obtained in large amounts.

In addition, using antibodies prepared against the protein coded by the DNAs of the present invention and the water-soluble muteins of the present invention or the amino acid adducts thereof, cancer or prostatic hypertrophy can be diagnosed by detecting the amount of the FGF receptors expressed in the cells.

When the water-soluble muteins of the present invention or the amino acid adducts thereof are used as pharmaceutical preparations, they can be safely given to warm-blooded mammals (such as humans, mice, rats, hamsters, rabbits, dogs and cats) parenterally or orally, in a powder form as they are, or as pharmaceutical compositions (such as injections, tablets, capsules, solutions and ointments) with pharmaceutically acceptable carriers, excipients and diluents.

The injections are prepared by conventional methods using, for example, physiological saline or aqueous solutions containing glucose or other auxiliary agents. The pharmaceutical compositions such as tablets and capsules can also be prepared in accordance with conventional methods.

When the water-soluble muteins of the present invention or the amino acid adducts thereof are given to mammals as the above-mentioned pharmaceutical compositions, the dosage is, for example, about 0.2 »g/kg to 0.2 mg/kg a day, and more preferably about 2 »g/kg to 0.2 mg/kg.

Further, when the cell cultivation of the cells into which the genes coding for the water-soluble muteins of the present invention or the amino acid adducts thereof are introduced should be accelerated, the FGF is preferably added to a culture medium so as to be contained in an amount of about 0.01 to 10 »g, and more preferably in an amount of about 0.1 to 10 »g per liter of medium.

When nucleotides, amino acids and so on are indicated by the abbreviations in this specification and drawings, the abbreviations adopted by IUPAC-IUB Commission on Biochemical Nomenclature or commonly used in the art are employed. For example, the following abbreviations are used. When the optical isomer is capable of existing with respect to the amino acids, the L-form is represented unless otherwise specified.
- DNA :: Deoxyribonucleic acid
- cDNA :: Complementary deoxyribonucleic acid
- A :: Adenine
- T :: Thymine
- G :: Guanine
- C :: Cytosine
- RNA :: Ribonucleic acid
- dATP :: Deoxyadenosine triphosphate
- dTTP :: Deoxythymidine triphosphate
- dGTP :: Deoxyguanosine triphosphate
- dCTP :: Deoxycytidine triphosphate
- ATP :: Adenosine triphosphate
- EDTA :: Ethylenediaminetetraacetic acid
- SDS :: Sodium dodecyl sulfate
- G or Gly :: Glycine
- A or Ala :: Alanine
- V or Val :: Valine
- L or Leu :: Leucine
- I or Ile :: Isoleucine
- S or Ser :: Serine
- T or Thr :: Threonine
- C or Cys :: Cysteine
- M or Met :: Methionine
- E or Glu :: Glutamic acid
- D or Asp :: Aspartic acid
- K or Lys :: Lysine
- R or Arg :: Arginine
- H or His :: Histidine
- F or Phe :: Phenylalanine
- Y or Tyr :: Tyrosine
- W or Trp :: Tryptophan
- P or Pro :: Proline
- N or Asn :: Asparagine
- Q or Gln :: Glutamine

The accession numbers and deposit dates of the transformants obtained in Examples described below are shown in Table 1. In Table 1, the IFO numbers indicate the deposition in the Institute for Fermentation, Osaka, Japan (IFO) and the FERM BP numbers indicate the deposition in the Fermentation Research Institute, the Agency of Industrial Science and Technology, the Ministry of International Trade and Industry, Japan (FRI) under the Budapest treaty.

**Table 1**

| Microorganism | IFO | FRI |
|---|---|---|
| E. coli MV1184/pTB1228 | IFO 15071 (July 24, 1990) | FERM BP-3041 (August 6, 1990) |
| E. coli MV1184 pTB1229 | IFO 15072 (July 24, 1990) | FERM BP-3042 (August 6, 1990) |
| E. coli MV1184 pTB1283 | IFO 15089 (September 13,1990) | FERM BP-3214 (December 26, 1990) |
| E. coli MV1184 pTB1284 | IFO 15090 (September 13,1990) | FERM BP-3215 (December 26, 1990) |
| E. coli MM294(DE3)/pLysS, pTB1289 | IFO 15091 (September 13,1990) | FERM BP-3216 (December 26, 1990) |
| E. coli MM294(DE3)/pLysS, pTB1290 | IFO 15092 (September 13,1990) | FERM BP-3217 (December 26, 1990) |
| CHO 1315-1023 | IFO 50326 (April 3, 1991) | FERM BP-3362 (April 16, 1991) |
| CHO 1316-172 | IFO 50327 (April 3, 1991) | FERM BP-3363 (April 16, 1991) |

The present invention will be described in more detail with the following Examples. It is understood of course that these Examples are not intended to limit the scope of the invention.

### Example 1

### (1) Preparation of Human Cancer Cell Line Kato III mRNA-Derived cDNA Library

mRNA was extracted from human cancer cell Line Kato III by using an mRNA separating kit (Fast Track, Invitrogen, USA). Using this mRNA as a template, a cDNA library was prepared by use of λphage vector λgt10 [T. V. Huynh et al., DNA Cloning, A Practical Approach, p.49, IRL Press, Oxford (1985)] according to the method of Watson and Jackson [C. J. Watson and J. F. Jackson, DNA Cloning, A Practical Approach, p.79, IRL Press, Oxford (1985)]. Starting from 10 »g of poly(A) RNA, the cDNA library whose host is E. coli C600, HflA (T. V. Huynh et al., the same as above) comprising about 1.5X10⁶ clones could be obtained.

### (2) Isolation of Phage Containing cDNA of Protein Having Human bFGF Receiving Action and Determination of DNA Nucleotide Sequence Thereof

Each of 10 agar plates was inoculated to about 1X10⁵ clones with the above phage cDNA library using E. coli C600, HflA as a host, and then the cDNA of each plate was transferred to 2 nitrocellulose filters (Millipore, HATF filter). Then, phage DNA was exposed for denaturation by lysing with 0.5 N NaOH solution and dried for fixation on the filters [T. Maniatis et al., Molecular Cloning, p.320, Cold Spring Harbor Laboratory (1982)].

On the other hand, an oligonucleotide having the following formula was chemically synthesized which was deduced from a portion (amino acid Nos. 529 to 541) of the amino acid sequence of the chicken bFGF receptor reported by P. Lee et al. [P. Lee et al., Science 7, 57 (1989)].
5'-ATTCTTATGCTTCCCGATCATQTTCATCATQTCCATQTC-3' Q: T/C
This oligonucleotide was reacted with T4 polynucleotide kinase (Takara Shuzo) in 50 »l of a reaction solution [oligonucleotide: 0.1 »g, 50 mM Tris-HCl (pH 8.0), 10 mM MgCl₂, 10 mM mercaptoethanol, 50 »Ci [γ-³²P]-ATP ( > 5000 Ci/mmole), 3 units of T4 polynucleotide kinase] at 37^{o}C for 1 hour to label the 5'-terminus of the oligonucleotide with ³²P.

The oligonucleotide labeled by the method described above was hybridized as a probe with each of the replica filters on which the DNA was fixed. The hybridization reaction was carried out at 35^{o}C for 16 hours in 10 ml of a solution of 5 X SSPE [180 mM NaCl, 10 mM NaH₂PO₄, 1 mM EDTA (pH 7.4)], 5 X Denhardt's, 0.1% SDS and 100 »g/ml denatured salmon sperm DNA. After completion of the reaction, the filters were washed with a solution of 5 X SSC (0.15 M NaCl, 0.015 M sodium citrate) and 0.1% SDS at room temperature for 30 minutes 3 times and further at 45^{o}C for 30 minutes twice [T. Maniatis et al., Molecular Cloning, p.309, Cold Spring Harbor Laboratory (1982)].

Radioautograms were taken from the washed filters and the radioautograms of the replica filters in sets of two filters were put together in layers to search the cell strains reactive to the probe.

Consequently, 3 cDNA clones reactive to the probe were obtained, and named λFRK1, λFRK2 and λFRK3, respectively. A portion of the nucleotide sequence of each cDNA portion was determined by the dideoxynucleotide synthetic chain termination method [J. Messing et al., Nucleic Acids Res. 9, 309 (1981)].

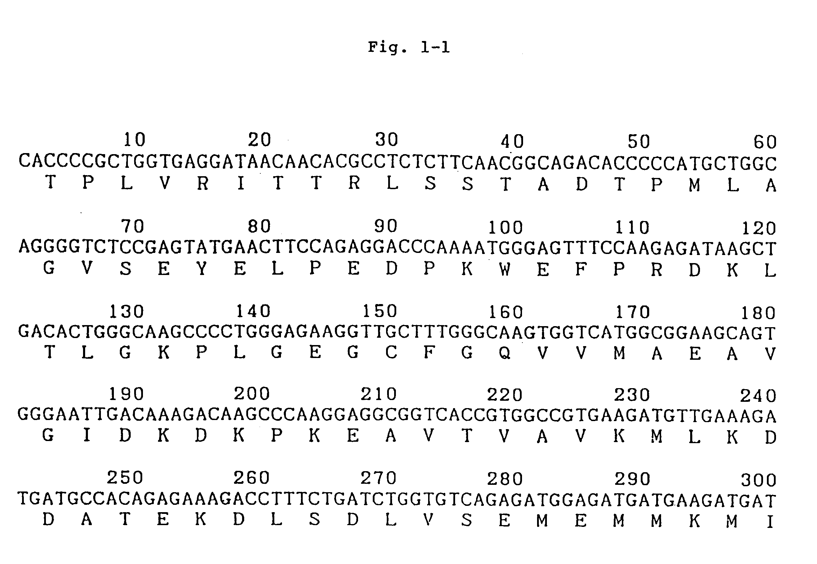

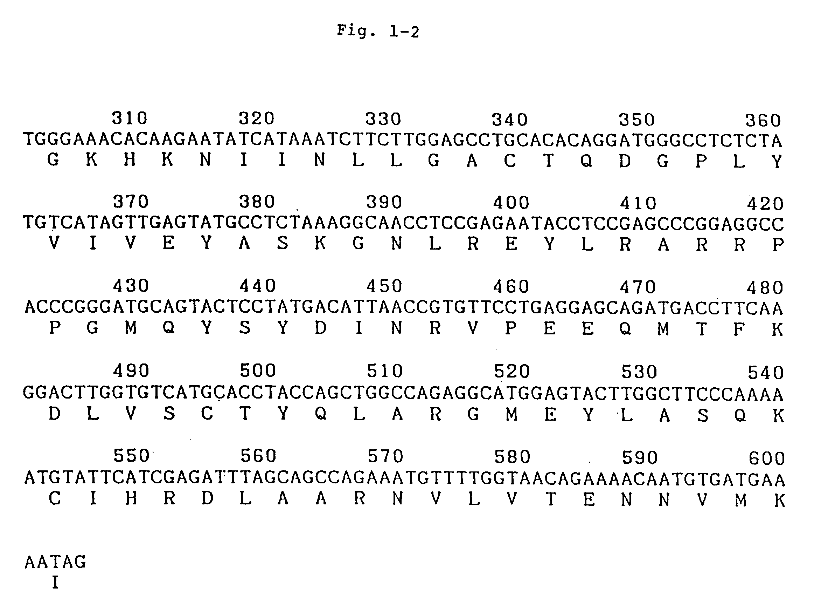

As a result, the amino acid sequence of a portion of the protein having human FGF receiving action could be determined. The nucleotide sequence and the amino acid sequence deduced therefrom are indicated by the sequences shown in Fig. 1. The amino acid sequence is very similar to that of the reported chicken bFGF receptor, which shows that this cDNA is one coding for the protein having action of receiving the human FGF proteins.

The protein obtained here is considered to lack the portion corresponding to about 190 amino acid residues from the C-terminus of the chicken FGF receptor [Science 7, 57 (1989)]. However, the protein is considered to contain at least the amino acid sequence corresponding to the central transmembrane sequence to the N-terminus.

### Example 2

Each of the cDNA portions of 3 cDNA clones λFRK1, λFRK2 and λFRK3 obtained in Example 1 was inserted into the multicloning sites of plasmids pUC118 and pUC119 (Takara Shuzo) to transform E. coli MV1184, thereby obtaining each of transformants E. coli MV1184/pTB1227, E. coli MV1184/pTB1228 (IFO 15071, FERM BP-3041) and E. coli MV1184/pTB1229 (IFO 15072, FERM BP-3042).

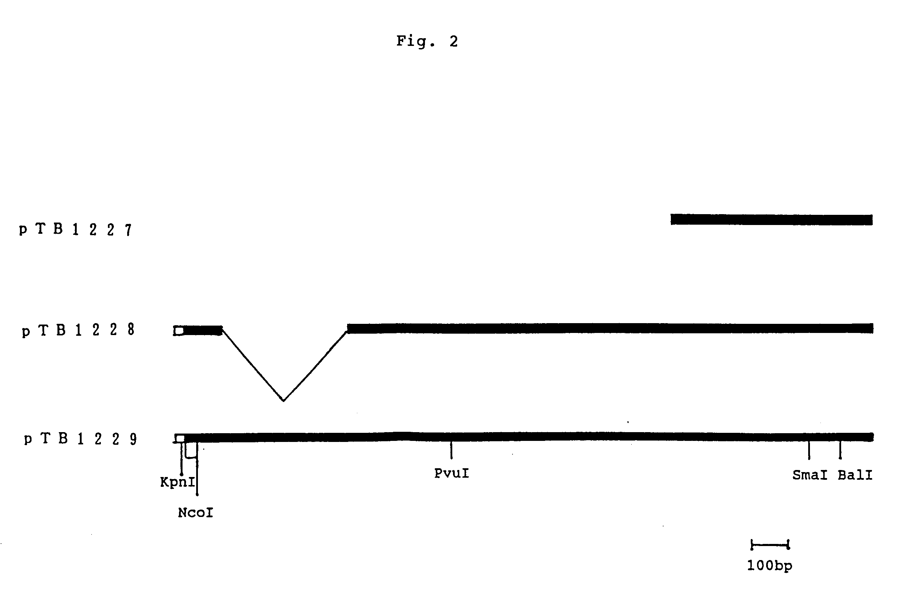

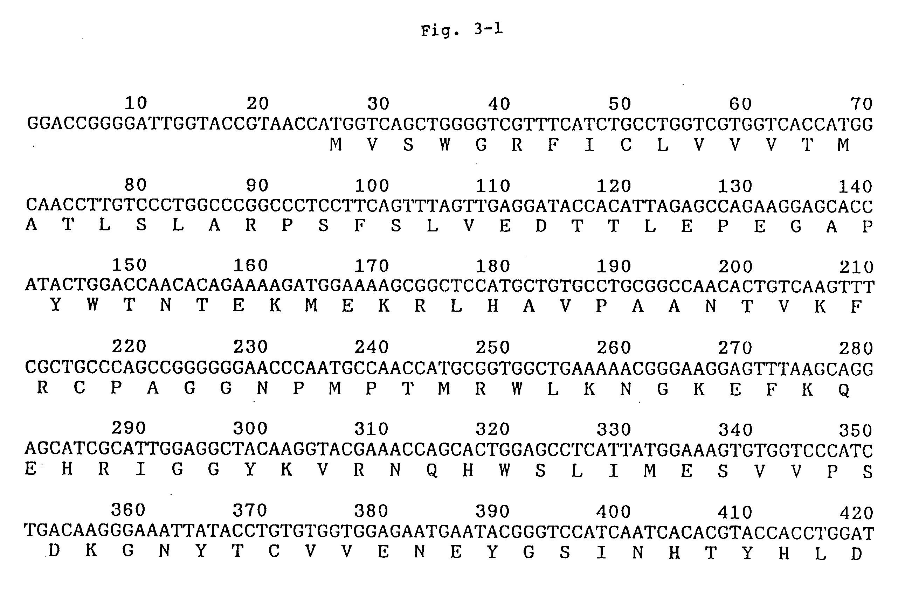

Fig. 2 shows restriction enzyme maps of the cDNA portions of plasmids pTB1227, pTB1228 and pTB1229 obtained from these transformants.

The cleavage maps obtained by digesting the cDNA portions of pTB1227, pTB1228 and pTB1229 with restriction enzymes are shown. The restriction enzymes used are KpnI, NcoI, PvuI, SmaI and BalI.

Referring to Fig. 2, open squares show non-coding regions, closed squares show coding regions, and V shows a deleted portion.

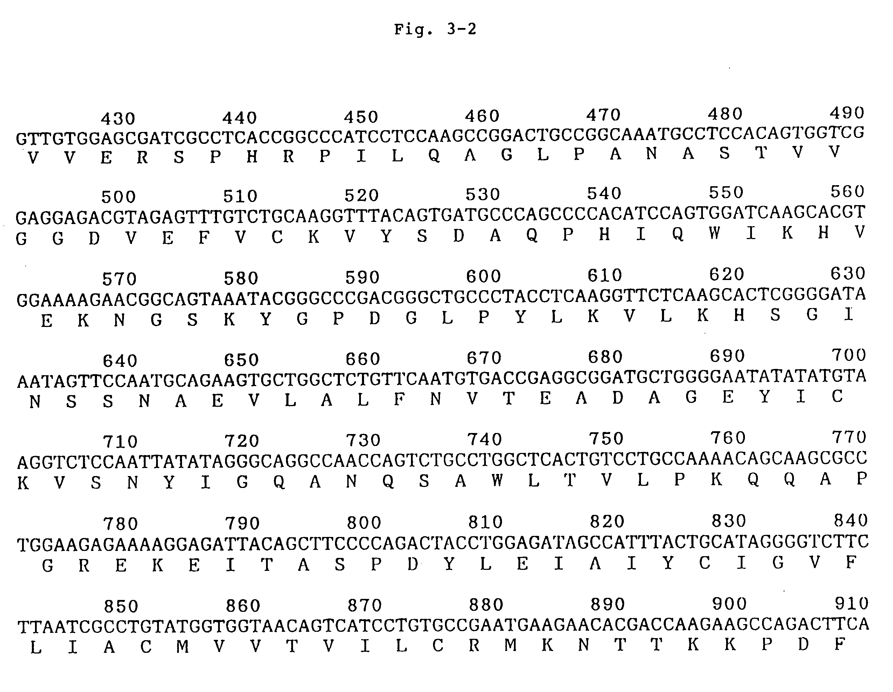

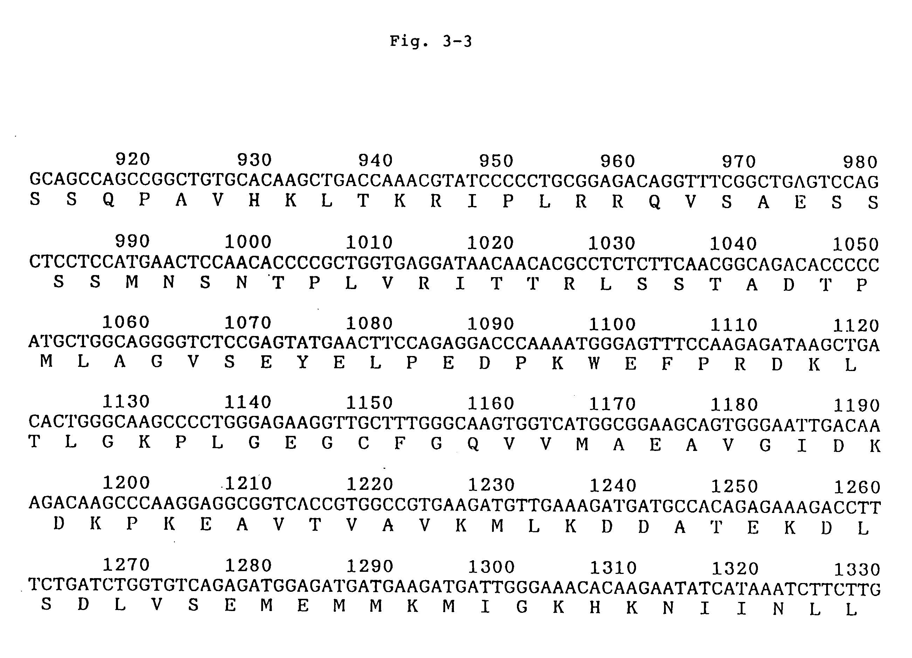

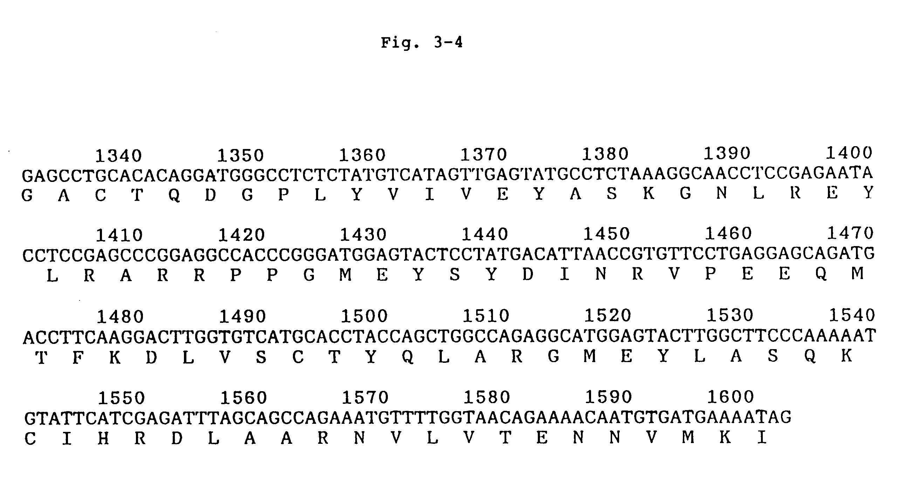

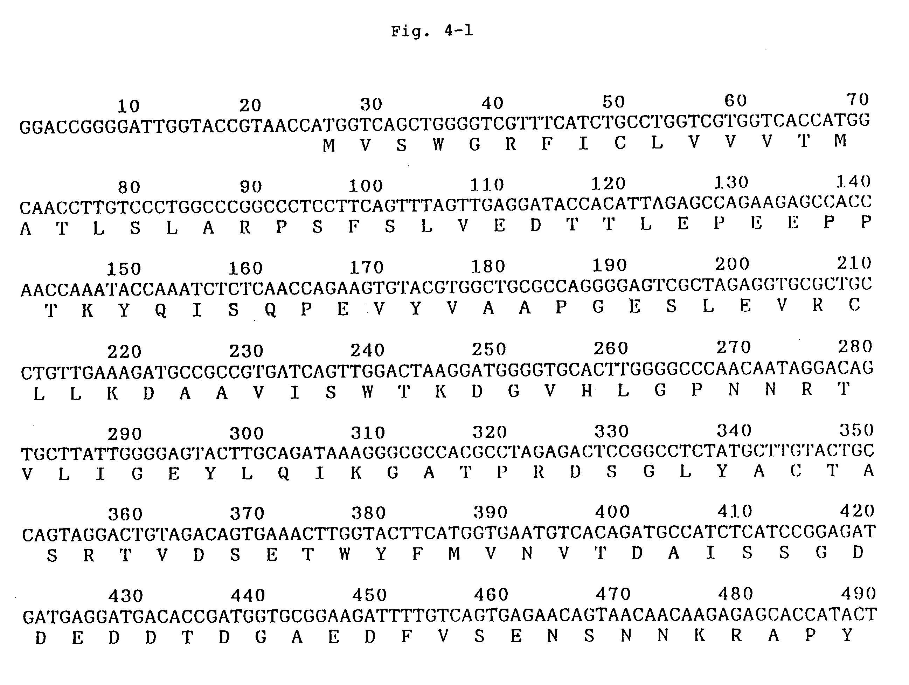

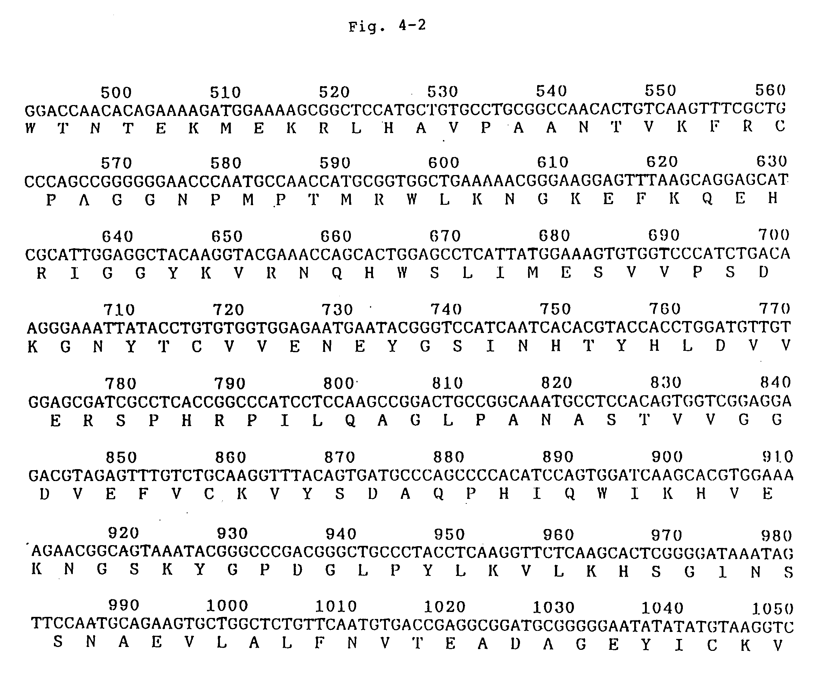

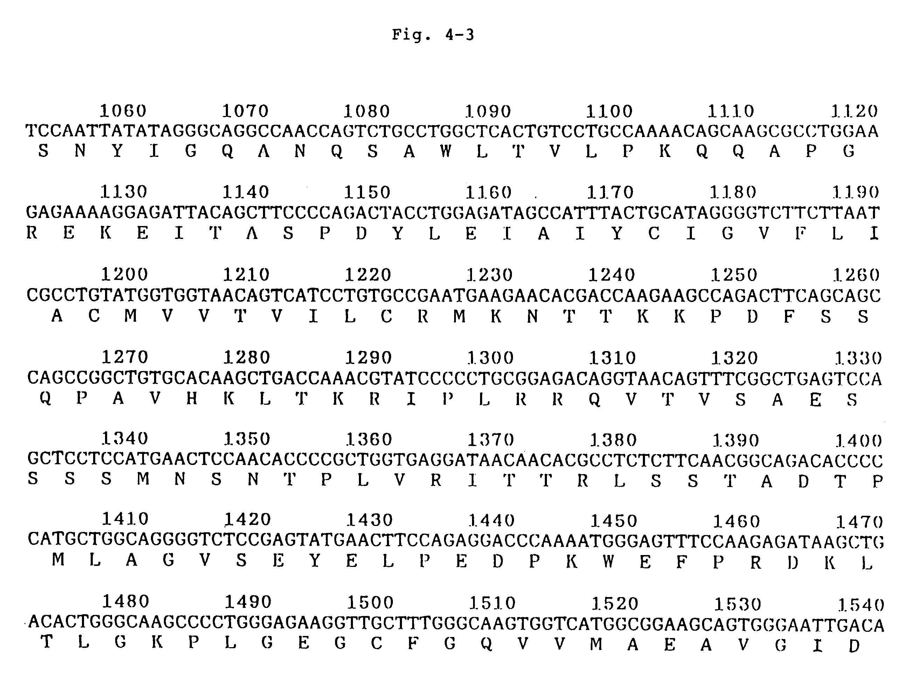

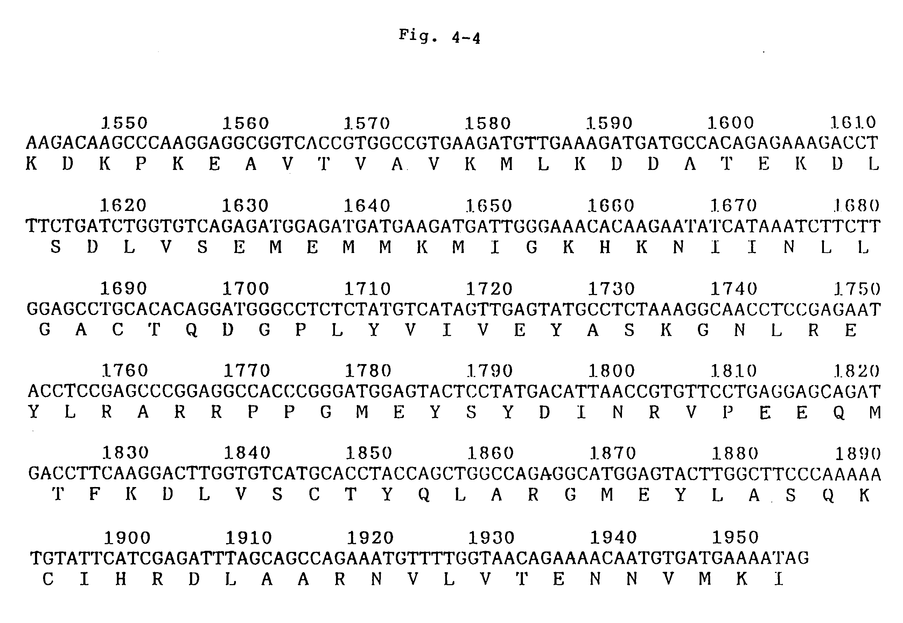

Further, the determination of the nucleotide sequences of these cDNAs proved that the nucleotide sequence of cDNA clone pTB1229 which was longest consisted of 1954 nucleotides. The nucleotide sequence thereof and the amino acid sequence for which it codes are indicated by the amino acid sequence shown in Fig. 4. The cDNA of pTB1228 is indicated by the nucleotide sequence shown in Fig. 3. This lacks the 134th to 478th nucleotides and 1309th and 1314th nucleotides of the nucleotide sequence shown in Fig. 4. Consequently, the amino acid sequence encoded lacks E³⁷ to K¹⁵¹, V⁴²⁹ and T⁴³⁰, and R¹⁵² is changed to G. Furthermore, in pTB1228, the 1029th G of the nucleotide sequence (Fig. 4) of the cDNA of pTB1229 is changed to T. In addition, the cDNA of pTB1227 was found to correspond to the 1395th to 1954th. Even when the nucleotide sequences of these cDNAs are translated to amino acids, no translation termination codons appear. For this reason, it is believed that the carboxyl terminus of the human FGF receptor cannot be obtained without further cloning the 3'-terminal side. When the translated amino acids are compared to those of the chicken FGF receptor, they have about 66% homology. From this comparison, the signal peptide is presumed to consist of 21 amino acids of M¹ to A²¹. Similarly, the transmembrane sequence is presumed to consist of I³⁷⁹ to C³⁹¹.

### Example 3

### (1) cDNA was synthesized by using the mRNA obtained in Example 1 described above and a cDNA synthesis kit (cDNA synthesis system, Plus, Amersham, U. K. ).

Primer 5'-TCAGAGATGGAGATGATGAAG-3' (21-mer) was synthesized based on the cDNA nucleotide sequence (1618-1638) obtained Example 2 described above, and the portion coding for the C-terminus of the FGF receptor was amplified to the cDNA obtained above together with oligo(dT)₁₂₋₁₈ (Amersham, U. K.) by the PCR (polymerase chain reaction) method. The amplified DNA fragment was cloned to the SmaI site of plasmid vector pUC118 (Takara Shuzo) to transform E. coli MV1184, thereby obtaining transformant E. coli MV1184/pTB1281.

The DNA sequence of plasmid pTB1281 obtained from this transformant was digested with restriction enzyme BamHI and KpnI. The length of the resulting cDNA was about 1 kbp.

Further, the nucleotide sequence of this cDNA was analyzed. As a result, this sequence was confirmed to contain a portion coding for V⁵³³ to the carboxyl terminus of the FGF receptor and a non-translation region on the 3'-terminal side.

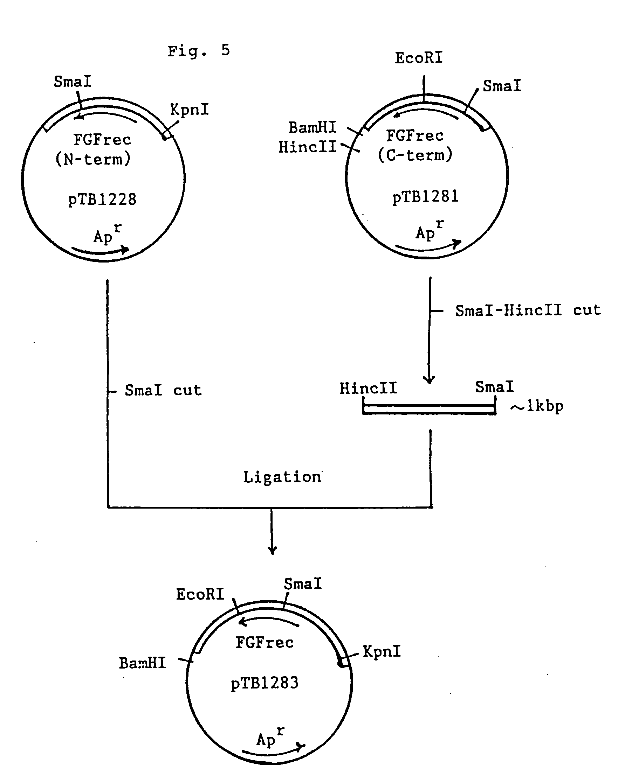

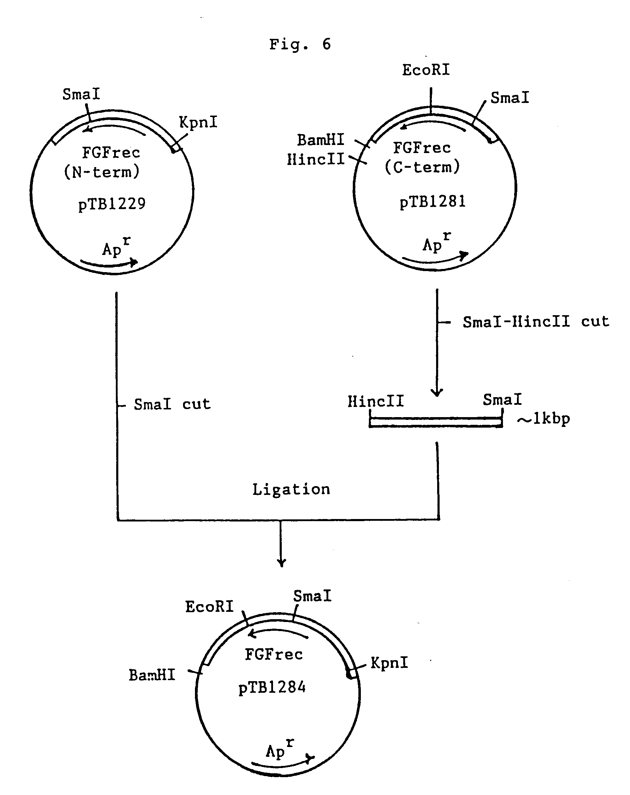

This cDNA nucleotide sequence overlaps with nucleotide No. 1618 and the succeeding nucleotides of the sequence shown in Fig. 3. Utilizing the recognition site of restriction enzyme SmaI existing in this portion, plasmid pTB1283 (Fig. 5) and plasmid pTB1284 (Fig. 6) having cDNA coding for the complete FGF receptor were constructed. Using these plasmid DNA, E. coli MV1184 was transformed to obtain two kinds of transformants E. coli MV1184/pTB1283 (IFO 15089, FERM BP-3214) and E. coli MV1184/pTB1284 (IFO 15090, FERM BP-3215).

The cDNA nucleotide sequence of plasmid pTB1284 is indicated by the nucleotide sequence shown in Fig. 7. The cDNA nucleotide sequence of plasmid pTB1283 is indicated by the nucleotide sequence shown in Fig. 8. This lacks the 134th to 478th nucleotides and 1309th to 1314th nucleotides of the nucleotide sequence shown in Fig. 7. Consequently, the amino acid sequence encoded lacks E³⁷ to K¹⁵¹, V⁴²⁹ and T⁴³⁰, and R¹⁵² is changed to G. Furthermore, the 1029th G of this cDNA is changed to T.

### (2) Expression of FGF Receptors in Animal Cells

### (a) Construction of Plasmids pTB1313 and pTB1314 for Expression of FGF Receptors

Two kinds of plasmids pTB1284 and pTB1283 obtained in the above item (1), each of which contains the whole structure region of the FGF receptor, were cleaved with restriction enzyme KpnI-BsmI. After reaction with T4 polymerase, BamHI linkers [CGGATCCG] were ligated to both terminal thereof, and a 2.6-kb or 2.2-kb DNA fragment was isolated.

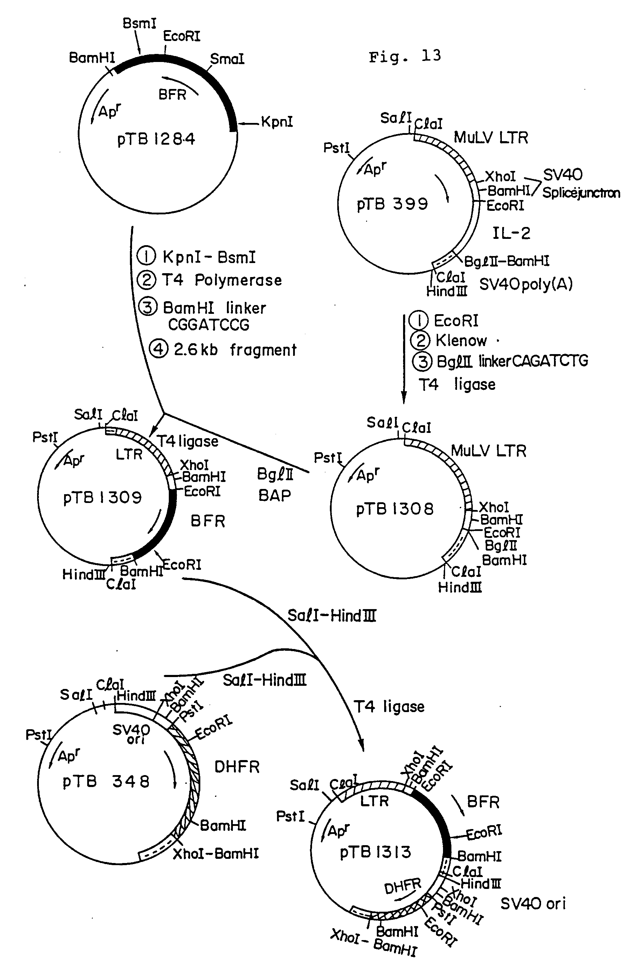

On the other hand, animal cell vector pTB1308 was obtained by cleaving plasmid pTB399 (Cell Struct. Funct. 12, 205-217 (1987)) with EcoRI to remove the IL-2 cDNA region thereof, ligating a BglII linker [CAGATCTG] thereto after Klenow fragment reaction, followed by cleavage with BglII, and cyclizing the resulting DNA fragment of about 3.8 kb by using T4 ligase. The above 2.6-kb or 2.2-kb fragment of the FGF receptor cDNA was inserted into the BglII cleavage site of pTB1308, and expression plasmid pTB1309 or pTB1310 which can express the FGF receptor cDNA in an animal cell under the control of Abelson mouse leukemia virus (MuLV) LTR was constructed. Each of these plasmids was further cleaved with SalI-HindIII, and an expression unit portion (promoter gene-poly(A) signal) was inserted into the SalI-HindIII site upstream from the SV40 promoter of plasmid pTB348 for expression of hamster-dihydrofolate reductase (DHFR) [Cell Struct. Funct. 12, 205 (1987)] to construct each of plasmids pTB1313 and pTB1314. Fig. 13 shows the construction of plasmid pTB1313.

### (b) Expression in Animal Cells

Monkey COS7 cells were inoculated in DMEM medium containing 10% fetal calf serum in a dish for tissue cultivation having a diameter of 6 cm, and the next day, the medium was exchanged for the same medium. After 4 hours, the cells were transfected with 10 »g of the DNA of plasmid pTB1313 or pTB1314 by the calcium phosphate method [Graham et al., Virology 52, 456 (1973)]. The next day, the transfected cells were added to DMEM medium containing 0.5% fetal calf serum, and cultivation was continued. After 48 hours, the culture solution and the cells were collected. The cells were washed twice with PBS, and then suspended in PBS. The suspension was treated with ultrasonication for a short time, followed by centrifugation at 15,000 rpm for 15 minutes to separate a supernatant from a cell residue. These culture solutions (0.5 ml, 5% trichloroacetic acid precipitation), cell extracts and the cell residues were analyzed by the Western blotting method after SDS-PAGE. As a primary antibody, rabbit polyclonal antibody was used which was obtained using a synthetic peptide (LVEDTTLEPE) as an antigen, the peptide corresponding to 27th to 36th of the amino acid sequence of the FGF receptor deduced from the FGF receptor cDNA.

As a result of the Western blotting, a product of about 140 to 150 kd was detected from the residue of the pTB1313-infected COS7 cells, and a product of about 100 kd was detected from the residue of the pTB1314-infected COS7 cells.

### Example 4

### (1) Construction of Expression System of Soluble Receptor in E. coli

Each of transformants E. coli MV1184/pTB1228 and E. coli MV1184/pTB1229 obtained in Example 2 described above was infected with helper phage KQ7, and a single stranded DNA was prepared from a culture medium. Using this single stranded DNA as a template, site-directed mutagenesis was carried out by use of two synthetic primers, (1)5'-AATGGCTGGATCCAGTTAGTCTGGGG-3' (26-mer) and (2)5'-GAAGGAGGGCCGCATATGGGACAAGGTTGC-3' (30-mer). This mutagenesis was conducted by using an in vitro mutagenesis system (Amersham, U. K.). As a result, plasmids pTB1285 and pTB1286 were obtained in each of which the 1152nd nucleotide of the nucleotide sequence of Fig. 7 was mutagenized to A by primer (1), a translation termination codon was introduced instead of Y³⁷⁶, and further, the recognition site of restriction enzyme NdeI was introduced into the 1155th to 1160th nucleotides. Then, plasmids pTB1287 and pTB1288 were obtained in each of which the 83rd to 87th nucleotides were substituted for ATATG, the codon of A²¹ was changed to the codon of M, and further, the recognition site of restriction enzyme NdeI was introduced into this portion.

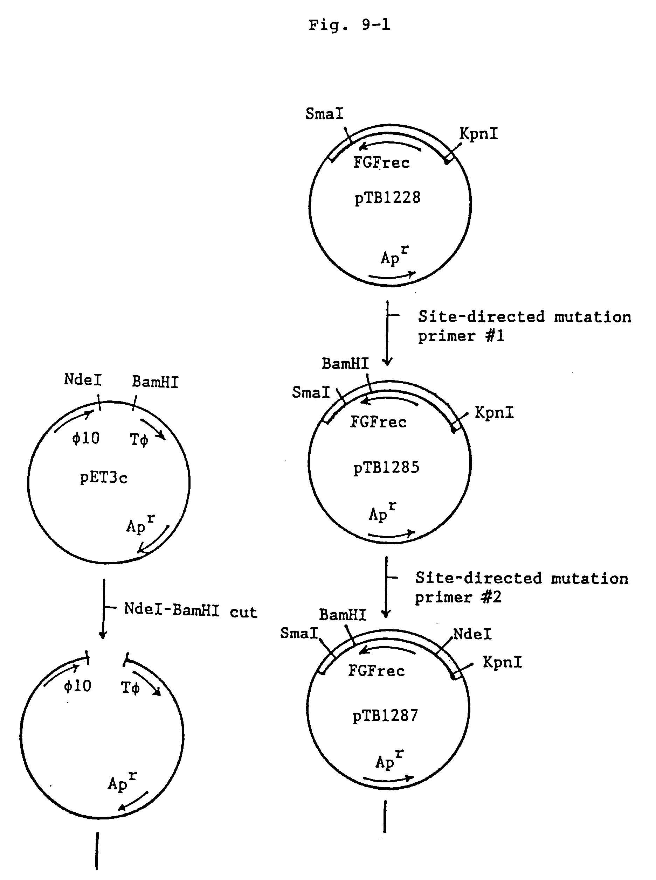

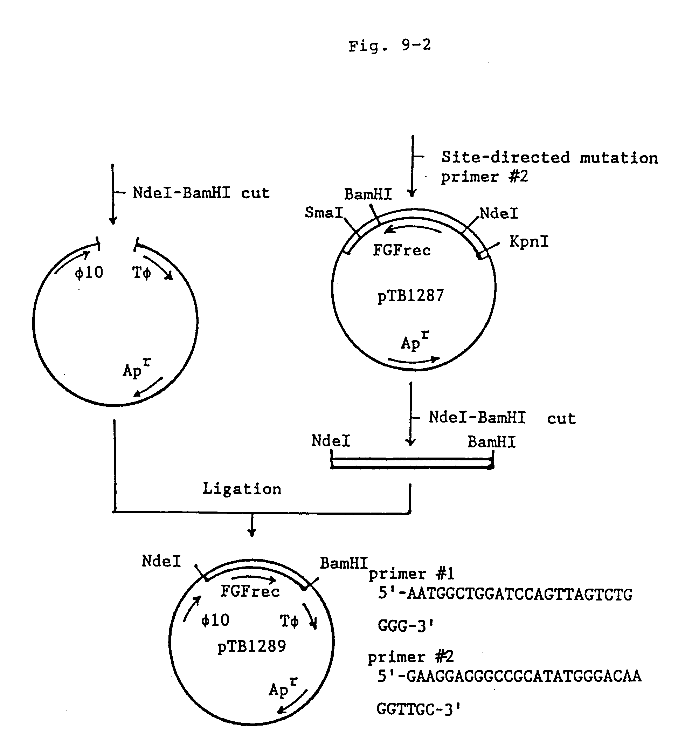

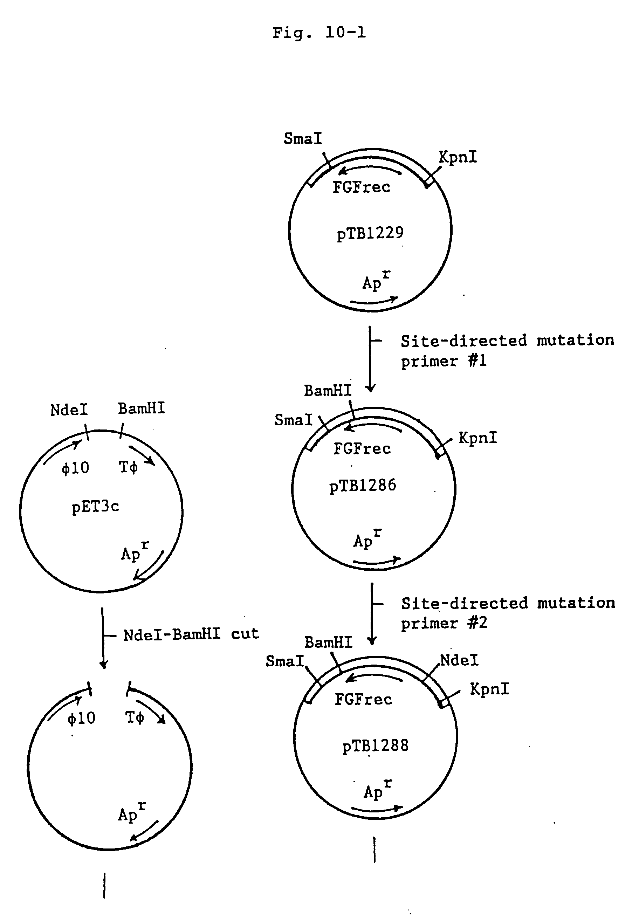

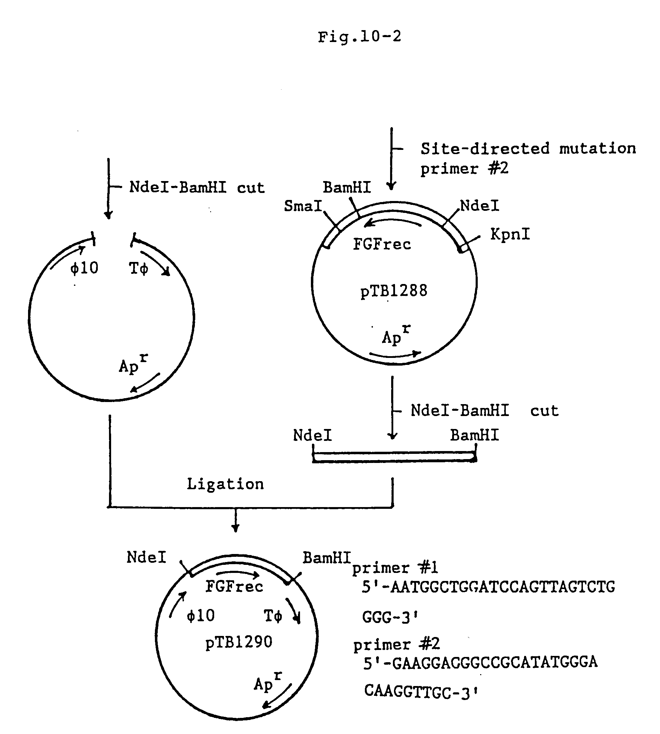

A fragment of 0.7 kbp or 1 kbp which was cut out from the DNA of each mutant with NdeI or BamHI was introduced downstream from the T7 promoter of plasmid pET3c to obtain plasmid pTB1289 or pTB1290. Fig. 9 shows the construction of plasmid pTB1289, and Fig. 10 shows the construction of plasmid pTB1290. E. coli MM294 (DE3)/pLysS was transformed using these plasmids, whereby transformants E. coli MM294 (DE3)/pLysS, pTB1289 (IFO 15091, FERM BP-3216) and E. coli MM294 (DE3)/pLysS, pTB1290 (IFO 15092, FERM BP-3217) expressing an extracellular domain of the FGF receptor were obtained.

### (2) Expression of Extracellular Domains of FGF Receptors in E. coli

Each of E. coli MM294 (DE3)/pLysS, pTB1289 and E. coli MM294 (DE3)/pLysS, pTB1290 was cultivated in LB medium and expression was induced with isopropyl-β-D-thiogalactoside. Then, all proteins of the cells were examined by SDS-PAGE. As a result, bands specific for the respective transformants were confirmed by Coomassie Blue staining. From the relative positional relations to molecular weight markers, these bands were considered to agree with the estimated molecular weight of the proteins encoded in the cDNAs, and the production of the extracellular domains of the FGF receptors in E. coli was confirmed. As to the amino acid sequences of the extracellular domains of the resulting FGF receptors, the amino acid sequence of the protein expressed by E. coli MM294 (DE3)/pLysS, pTB1289 is indicated by the sequence shown in Fig. 11, and the amino acid sequence of the protein expressed by E. coli MM294 (DE3)/pLysS, pTB1290 is indicated by the sequence shown in Fig. 12.

### (3) Expression of Extracellular Domains of FGF Receptors in Animal Cells

### (a) Construction of Expression Plasmids pTB1315 and pTB1316

Two kinds of plasmids pTB1286 and pTB1285 obtained in the above (1), each of which has cDNA coding for the extracellular domain of the FGF receptors, were cleaved with restriction enzymes BstEII-BamHI to obtain 1.1-kb and 0.75-kb DNA fragments. On the other hand, plasmid pTB1309 for expression of the FGF receptors constructed in Example 3 (2) described above was cleaved with the same restriction enzymes, and replaced with each of the above-mentioned 1.1-kb and 0.75-kb fragments to construct expression plasmids pTB1311 and pTB1312.

pTB1311 and pTB1312 are plasmids which can express the cell domains of two kinds of FGF receptors under the control of MuLV LTR.

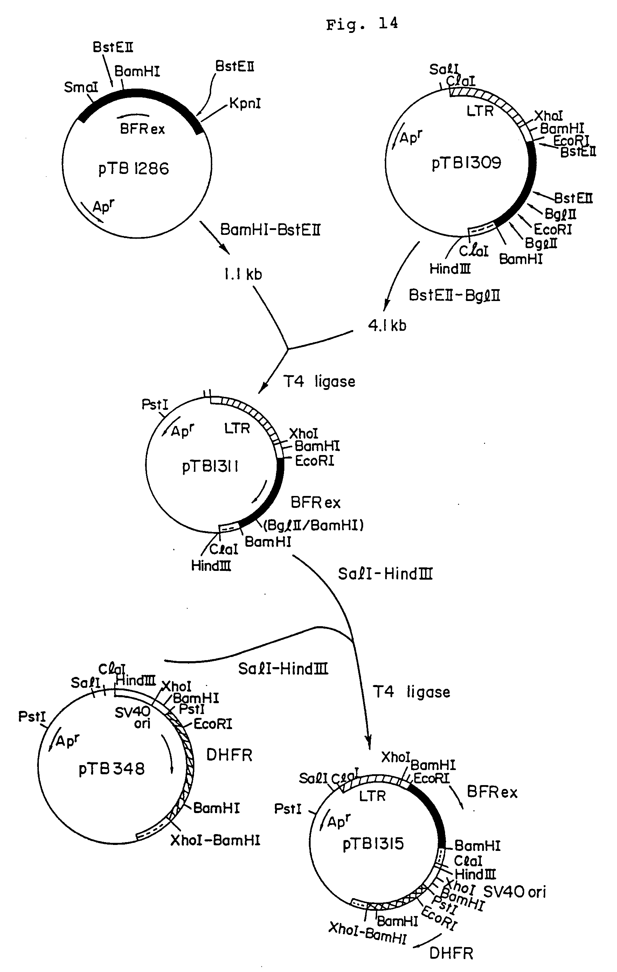

Further, each of these plasmids was cleaved with SalI-HindIII to obtain an expression unit portion, which was inserted into plasmid pTB348 for expression of DHFR, using the SalI-HindIII fragment similarly with Example 3 (2) described above, thereby constructing plasmids pTB1315 and pTB1316. Fig. 14 shows the construction of plasmid pTB1315.

### (b) Expression in Animal Cells

Monkey COS7 cells were transfected with plasmid pTB1313 or pTB1314 similarly with Example 3 (2) described above. For culture supernatants, cell extracts and cell residues, the infected cells were analyzed by western blotting. As a result, products of about 50 to 60 kd and about 35 to 50 kd were detected in the pTB1315-infected and pTB1316-infected COS7 cell culture solutions, respectively.

The water-soluble muteins of the animal proteins having action of receiving FGF proteins are useful as therapeutic agents or to enhance cell proliferation activity by giving reactivity with the FGFs to cells having no FGF dependence.

### Example 5

### Purification of Extracellular Domain of FGF Receptor Produced in E. coli

E. coli MM294 (DE3)/pLysS, pTB1289 was cultivated in a medium containing 35 »g/ml ampicillin and 10 »g/ml chloramphenicol at 37^{o}C. When the turbidity reached Klett 120, isopropyl-β-D-thiogalactoside was added thereto to a final concentration of 0.5 mM, and cultivation was further continued for 2 hours. The cells were collected by centrifugation, and washed with phosphate buffered saline (PBS) cooled with ice. Then, the cells were collected again and stored at -20^{o}C until they were used.

The E. coli cells collected from 1 liter of the culture were suspended in 25 ml of an ice-cooled solution [7 M urea, 0.1 M Tris-HCl (pH 8) and 10 mM dithiothreitol (DTT)], and allowed to stand in ice for 1 hour.

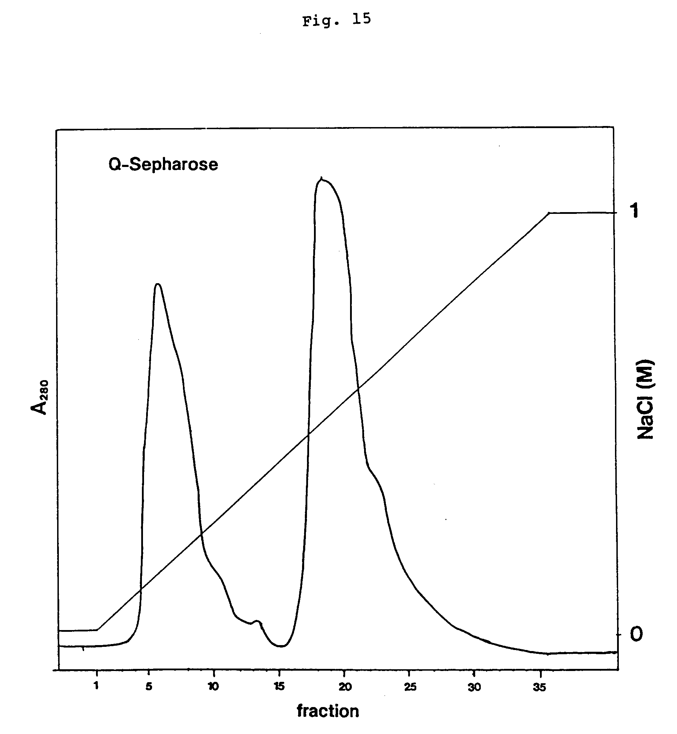

After this solution was centrifuged at 17,000 rpm for 1 hour, the resulting supernatant was subjected to a Q-Sepharose column (2.5 cm in diameter by 5 cm) equilibrated with a buffer (7 M urea, 0.1 M Tris-HCl (pH 8) and 1 mM DTT). After washing with the buffer used to equilibrate the column, the column was eluted with a linear gradient of 0 to 1 M NaCl at a flow rate of 1 ml/minute for 300 minutes, and fractionation was carried out every 8 minutes (Fig. 15). Each fraction was analyzed by the Western blotting method. As a result, it was observed that FGF receptor extracellular domain BFR-2ex was eluted in fraction Nos. 10 and 11 (the amino acid sequence thereof is shown in Fig. 11).

These eluted fractions of the Q-Sepharose column were further subjected to a Sephacryl S-200 column (2.5 cm in diameter by 100 cm) equilibrated with a buffer (7 M urea, 0.1 M Tris-HCl (pH 8), 0.3 M NaCl and 2 mM DTT) to conduct gel filtration at a flow rate of 40 ml/hour. The results thus obtained revealed that FGF receptor extracellular domain BFR-2ex was eluted at the position corresponding to a molecular weight of about 32,000. The resulting fraction exhibited a single band by SDS-polyacrylamide gel electrophoresis and Coomassie Blue staining.

### Example 6

### Establishment of CHO Cell Strains Producing Extracellular Domains of FGF Receptors

Expression plasmids pTB1315 and pTB1316 described in Example 4, (3), (a) were each introduced into CHO dhfr⁻ cells [Urlanb et al., Proc. Natl. Acad. Sci. USA 77, 4216 (1980)] by the calcium phosphate method [Graham et al., Virology 52, 456 (1973)].

After two days, the medium was exchanged for a selection medium (DMEM containing 10% dialyzed fetal calf serum and 35 »g/ml proline), and cultivation was continued to obtain transformants which proliferated as dhfr⁺.

These CHO dhfr⁺ cells were cloned by the limiting dilution method, and 24 clones were established for the respective CHO cells obtained by transfecting the respective plasmids.

Then, each CHO dhfr⁺ clone was first cultivated in DMEM (containing 5% fetal calf serum and 35 »g/ml proline) containing 0.1 »M methotrexate (MTX), and cells which acquired MTX resistance by gene amplification were selected. Thereafter, 1 »M MTX-resistant cells and 10 »M MTX-resistant cells were selected in turn.

For the respective CHO dhfr⁺ cell clones thus obtained and the MTX-resistant cell strains thereof, FGF receptor extracellular domains secreted in the culture solutions were detected by using the Western blotting method, thereby screening the produced cell strains. Namely, each cell strain proliferated to a confluent of 80% on a 24-well plate was cultivated in DMEM/Ham's medium (1:1) containing 0.5% fetal calf serum and 10 »g/ml insulin for 3 days. After SDS-PAGE, 0.5 ml of that culture solution [5% trichloroacetic acid (TCA) precipitation] was submitted to the Western blotting method by using the rabbit polyclonal antibody described in Example 3, (b) as a primary antibody.

The results revealed that CHO 1315-115, CHO 1315-123 and CHO 1315-1023 (IFO 50326, FERM BP-3362) cells obtained as 1 »M or 10 »M MTX-resistant cells by transfecting pTB1315 produced and secreted FGF receptor cell domains (BFR-1ex) of 60 to 70 kDa (the amino acid sequence thereof is shown in Fig. 12). Further, the results revealed that CHO 1316-161, CHO 1316-172 (IFO 50327, FERM BP-3363) and CHO 1316-1053 cells obtained as 1 »M or 10 »M MTX-resistant cells by transfecting pTB1316 produced and secreted FGF receptor extracellular domains (BFR-2ex) of 40 to 50 kDa.

The BFR-1ex- or BFR-2ex-producing CHO cell line was cultivated, adding ³⁵S-methionine (1000 Ci/mmole, 50 »Ci/ml) thereto, in the presence or absence of tunicamycin (5 »g/ml), and the ³⁵S-methionine-labeled protein in the culture solution was subjected to immune precipitation by using the above-mentioned rabbit polyclonal antibody, followed by analysis by SDS-PAGE. As a result, it was suggested that the 46-kDa protein was glycosylated to a 70-kDa protein for BFR-1ex produced by these transformant CHO cells, and that the 35-kDa protein was glycosylated to a 45-kDa protein for BFR-2ex.

The following references, which are referred to for their disclosures at various points in this application, are incorporated herein by reference.
European Patent Publication No. 237,966
European Patent Publication No. 281,822
European Patent Publication No. 326,907
European Patent Publication No. 394,951.
Nucleic Acids Research 18, 1906 (1990)
The EMBO Journal 9, 2685 (1990)
Molecular and Cellular Biology 8, 5541-5544 (1988)
Proc. Natl. Acad. Sci. USA 87, 4378-4382 (1990)
Science 245, 57-60 (1989)
Genetic Engineering, Academic Press, p.31-50 (1983)
Genetic Engineering: Principles and Methods, Plenum Press, vol. 3, p.1-32 (1981)
Catalogue of Cell Lines & Hybridomas, 5th edition (1985), published by ATCC
Biochemistry 18, 5294 (1979)
Molecular and Cellular Biology 2, 161 (1982)
Molecular and Cellular Biology 3, 280 (1983)
Gene 2, 95 (1977)
Gene 4, 121 (1978)
Gene 19, 259 (1982)
Methods in Enzymology 153, 3-11 (1987)
Biochemical and Biophysical Research Communication 112, 678 (1983)
Molecular Cloning, Cold Spring Laboratory, p.239 (1982)
Proc. Natl. Acad. Sci. USA 60, 160 (1968)
Nucleic Acids Research 9, 309 (1981)
Journal of Molecular Biology 120, 517, (1978)
Journal of Molecular Biology 41, 459 (1969)
Genetics 39, 440 (1954)
Gene 24, 255 (1983)
Journal of Biochemistry 95, 87 (1984)
Molecular Cloning, Cold Spring Harbor Laboratory, p.249 (1982).
Proc. Natl. Acad. Sci. USA 74, 560 (1977)
Nucleic Acids Research 1, 1513 (1979)
Proc. Natl. Acad. Sci. USA, 69, 2110 (1972)
Gene, 17, 107 (1982)
Molecular & General Genetics, 168, 111 (1979)
Proc. Natl. Acad. Sci. USA, 75, 1929 (1978)
Virology, 52, 456 (1973)
Journal of Experiments in Molecular Genetics, 431-433, Cold Spring Harbor Laboratory, New York, 1972
Proc. Natl. Acad. Sci. USA, 77, 4505 (1980)
Science, 122, 501 (1952)
Virology, 8, 396 (1959)
Journal of the American Medical Association, 199, 519 (1967)
Proceeding of the Society for the Biological Medicine, 73, 1 (1950)
DNA Cloning, A Practical Approach, p.49, IRL Press, Oxford (1985)
DNA Cloning, A Practical Approach, p.79, IRL Press, Oxford (1985)
Molecular Cloning, p.320, Cold Spring Harbor Laboratory (1982)
Science 7, 57 (1989)
Molecular Cloning, p.309, Cold Spring Harbor Laboratory (1982)
Cell Struct. Funct. 12, 205-217 (1987)

## Claims (Claims for the following Contracting State(s): AT, BE, CH, DE, DK, FR, GB, GR, IT, LI, LU, NL, SE)

1. A water-soluble mutein of fibroblast growth factor (FGF) receptor which mutein lacks either (1) an amino acid sequence corresponding to the transmembrane domain of the mature FGF receptor or (2) amino acid residues from the C-terminus to the transmembrane domain of the mature FGF receptor.

2. The water-soluble mutein according to claim 1 which contains the amino acid sequence shown in Fig. 11.

3. The water-soluble mutein according to claim 1 which contains the amino acid sequence shown in Fig. 12

4. A recombinant DNA having a nucleotide sequence coding for the water-soluble mutein according to any of claims 1-3.

5. A vector containing the recombinant DNA according to claim 4.

6. A transformant transformed with the vector according to claim 5.

7. The transformant according to claim 6, which has the characteristics of E. coli MM294 (DE3)/pLysS, pTB1289.

8. The transformant according to claim 6, which has the characteristics of E. coli MM294 (DE3)/pLysS, pTB1290.

9. A process for producing the water-soluble mutein according to claim 1 which comprises cultivating the transformant claimed in claim 6 in a culture medium.

## Claims (Claims for the following Contracting State(s): ES)

1. A process for producing water-soluble mutein of fibroblast growth factor (FGF) receptor which mutein lacks either (1) an amino acid sequence corresponding to the transmembrane domain of the mature FGF receptor or (2) amino acid residues from the C-terminus to the transmembrane domain of the mature FGF receptor which process comprises cultivating a transformant transformed with a vector containing a recombinant DNA having a nucleotide sequence coding for the above water-soluble mutein in a culture medium.

2. The process according to claim 1, wherein the water-soluble mutein contains the amino acid sequence shown in Fig. 11.

3. The process according to claim 1, wherein the water-soluble mutein contains the amino acid sequence shown in Fig. 12.

4. A process for producing a recombinant DNA having a nucleotide sequence coding for the water-soluble mutein as defined in any of claims 1-3, which process comprises isolating RNA coding for the mature protein.

5. A process for producing a vector containing the recombinant DNA as defined in claim 4, which process comprises ligating the DNA in the vehicle.

6. A process for producing a transformant transformed with the vector as defined in claim 5, which process comprises transforming a host cell with the vector.

7. The process according to claim 6, wherein the produced transformant has the characteristics of E. coli MM294 (DE3)/pLysS, pTB1289.

8. The process according to claim 6, wherein the produced transformant has the characteristics of E. coli MM294 (DE3)/pLysS, pTB1290.

## Patentansprüche (Patentansprüche für folgende(n) Vertragsstaat(en): AT, BE, CH, DE, DK, FR, GB, GR, IT, LI, LU, NL, SE)

1. Wasserlösliches Mutein des Rezeptors für den Fibroblasten-Wachstumsfaktor (FGF), wobei dem Mutein entweder (1) eine Aminosäuresequenz, die der Transmembrandomäne des reifen FGF-Rezeptors entspricht, oder (2) Aminosäurereste vom C-Terminus der Transmembrandomäne des reifen FGF-Rezeptors fehlen.

2. Wasserlösliches Mutein gemäß Anspruch 1, das die in Fig. 11 gezeigte Aminosäuresequenz enthält.

3. Wasserlösliches Mutein gemäß Anspruch 1, das die in Fig. 12 gezeigte Aminosäuresequenz enthält.

4. Rekombinante DNA, die eine Nucleotidsequenz aufweist, die für das wasserlösliche Mutein gemäß einem der Ansprüche 1-3 codiert.

5. Vektor, der die rekombinante DNA gemäß Anspruch 4 enthält.

6. Transformante, die mit dem Vektor gemäß Anspruch 5 transformiert ist.

7. Transformante gemäß Anspruch 6, die die Merkmale von *E*. *coli* MM294 (DE3)/pLysS, pTB1289 aufweist.

8. Transformante gemäß Anspruch 6, die die Merkmale von *E*. *coli* MM294 (DE3)/pLysS, pTB1290 aufweist.

9. Verfahren zur Herstellung des wasserlöslichen Muteins gemäß Anspruch 1, das das Kultivieren der Transformante gemäß Anspruch 6 in einem Kulturmedium umfaßt.

## Patentansprüche (Patentansprüche für folgende(n) Vertragsstaat(en): ES)

1. Verfahren zur Herstellung eines wasserlöslichen Muteins des Rezeptors für den Fibroblasten-Wachstumsfaktor (FGF), wobei dem Mutein entweder (1) eine Aminosäuresequenz, die der Transmembrandomäne des reifen FGF-Rezeptors entspricht, oder (2) Aminosäurereste vom C-Terminus der Transmembrandomäne des reifen FGF-Rezeptors fehlen, wobei das Verfahren das Kultivieren einer Transformante, die mit einem Vektor transformiert ist, der eine rekombinante DNA mit einer Nucleotidsequenz, die für das obige wasserlösliche Mutein codiert, enthält, in einem Kulturmedium umfaßt.

2. Verfahren gemäß Anspruch 1, wobei das wasserlösliche Mutein die in Fig. 11 gezeigte Aminosäuresequenz enthält.

3. Verfahren gemäß Anspruch 1, wobei das wasserlösliche Mutein die in Fig. 12 gezeigte Aminosäuresequenz enthält.

4. Verfahren zur Herstellung einer rekombinanten DNA, die eine Nucleotidsequenz aufweist, die für das wasserlösliche Mutein gemäß einem der Ansprüche 1-3 codiert, wobei das Verfahren das Isolieren von RNA, die für das reife Protein codiert, umfaßt.

5. Verfahren zur Herstellung eines Vektors, der die rekombinante DNA gemäß Anspruch 4 enthält, wobei das Verfahren das Ligasieren der DNA in den Träger umfaßt.

6. Verfahren zur Herstellung einer Transformante, die mit dem Vektor gemäß Anspruch 5 transsformiert ist, wobei das Verfahren das Transformieren einer Wirtszelle mit dem Vektor umfaßt.

7. Verfahren gemäß Anspruch 6, wobei die hergestellte Transformante die Merkmale von *E*. *coli* MM294 (DE3)/pLysS, pTB1289 aufweist.

8. Verfahren gemäß Anspruch 6, wobei die hergestellte Transformante die Merkmale von *E*. *coli* MM294 (DE3)/pLysS, pTB1290 aufweist.

## Revendications (Revendications pour l'(les) Etat(s) contractant(s) suivant(s): AT, BE, CH, DE, DK, FR, GB, GR, IT, LI, LU, NL, SE)

1. Protéine mutée hydrosoluble dérivée du récepteur du facteur de croissance des fibroblastes (FCF), à laquelle protéine mutée il manque :
a) soit une séquence d'acides aminés correspondant au domaine trans-membranaire du récepteur mature du FCF,
b) soit des résidus d'acides aminés, à partir de l'extrémité carboxy-terminale du domaine trans-membranaire du récepteur mature du FCF.

2. Protéine mutée hydrosoluble conforme à la revendication 1, qui comporte la séquence d'acides aminés représentée sur la figure 11.

3. Protéine mutée hydrosoluble conforme à la revendication 1, qui comporte la séquence d'acides aminés représentée sur la figure 12.

4. ADN recombiné, comportant une séquence de nucléotides codant une protéine mutée hydrosoluble conforme à l'une des revendications 1 à 3.

5. Vecteur contenant un ADN recombiné conforme à la revendication 4.

6. Cellule transformée avec un vecteur conforme à la revendication 5.

7. Cellule transformée conforme à la revendication 6, qui possède les caractéristiques d'E. coli MM294 (DE3)/pLysS, pTB 1289.

8. Cellule transformée conforme à la revendication 6, qui possède les caractéristiques d'E. coli MM294 (DE3)/pLysS, pTB 1290.

9. Procédé de préparation d'une protéine mutée hydrosoluble conforme à la revendication 1, qui comporte le fait de cultiver, dans un milieu de culture, une cellule transformée conforme à la revendication 6.

## Revendications (Revendications pour l'(les) Etat(s) contractant(s) suivant(s): ES)

1. Procédé de préparation d'une protéine mutée hydrosoluble dérivée du récepteur du facteur de croissance des fibroblastes (FCF), à laquelle protéine mutée il manque :
a) soit une séquence d'acides aminés correspondant au domaine trans-membranaire du récepteur mature du FCF,
b) soit des résidus d'acides aminés, à partir de l'extrémité carboxy-terminale du domaine trans-membranaire du récepteur mature du FCF, lequel procédé comporte le fait de cultiver, dans un milieu de culture, une cellule transformée avec un vecteur qui contient un ADN recombiné, comportant une séquence de nucléotides codant la protéine mutée hydrosoluble mentionnée ci-dessus.

2. Procédé conforme à la revendication 1, dans lequel la protéine mutée hydrosoluble comporte la séquence d'acides aminés représentée sur la figure 11.

3. Procédé conforme à la revendication 1, dans lequel la protéine mutée hydrosoluble comporte la séquence d'acides aminés représentée sur la figure 12.

4. Procédé de préparation d'un ADN recombiné, comportant une séquence de nucléotides codant une protéine mutée hydrosoluble définie dans l'une des revendications 1 à 3, lequel procédé comporte le fait d'isoler l'ARN codant la protéine mature.

5. Procédé de production d'un vecteur contenant un ADN recombiné défini dans la revendication 4, lequel procédé comporte le fait de ligaturer l'ADN dans le vecteur.

6. Procédé de production d'une cellule transformée avec un vecteur défini dans la revendication 5, lequel procédé comporte le fait de transformer une cellule hôte avec le vecteur.

7. Procédé conforme à la revendication 6, dans lequel la cellule transformée produite possède les caractéristiques d'E. coli MM294 (DE3)/pLysS, pTB 1289.

8. Procédé conforme à la revendication 6, dans lequel la cellule transformée produite possède les caractéristiques d'E. coli MM294 (DE3)/pLysS, pTB 1290.
